# EUROPEAN PATENT APPLICATION

(11) **EP 4 186 914 A1**
(43) Date of publication of application: **31.05.2023**
(21) Application number: 21211482.1
(22) Date of filing: 30.11.2021
(51) Int. Cl.: C07H 1/00, C07C 321/06, C07H 19/073, C07H 21/04

(54) **METHOD FOR PRODUCING POLYNUCLEOTIDES**

(71) Applicant: ATDBio Limited, Hengoed CF81 7TS (GB)
(72) Inventor: BROWN, Tom, Oxford (GB); COX, Owen, Oxford (GB)
(74) Representative: J A Kemp LLP

(57) **Abstract**

Provided herein is a method of producing a polynucleotide or an analogue or derivative thereof, comprising providing a support-attached nucleoside, nucleotide, polynucleotide or derivative or analogue thereof, which is a compound of formula (I) as defined herein and attached to a solid support at Ⓐ, 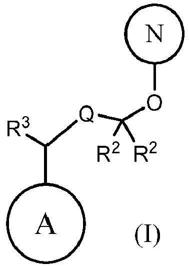 (ii) increasing the chain length of the support-attached nucleoside, nucleotide, polynucleotide or derivative or analogue thereof by introducing one or more additional nucleoside units, nucleotide units and/or analogues or derivatives thereof; and (iii) cleaving the resulting polynucleotide from the solid support by photo-illuminating the compound of formula (I). Also provided are associated compounds, uses, and polynucleotides obtainable by such methods.

## Description

### Field of the invention

The present invention relates to methods of producing a polynucleotide or an analogue or derivative thereof. The present invention also relates to compounds for use in such methods and to methods of sequencing using such polynucleotides. The present invention further relates to a solid support column comprising such compounds for solid-phase polynucleotide, the use of such compounds for synthesising polynucleotides or derivatives or analogues thereof, and polynucleotides or derivatives or analogues thereof obtainable by such methods.

### Background of the invention

Polynucleotides are biopolymers comprising nucleotide monomer units, and have applications in many different fields, including therapeutics, diagnostics and research. Polynucleotides with customised nucleotide sequences can be synthesised *in vitro,* for example using solution-phase or solid-phase synthetic methods.

Solid-phase chemical synthesis was developed in the 1960s and has a number of advantages over solution-phase synthesis. For example, large excesses of solution-phase reagents can be used to drive reactions quickly to completion; impurities and excess reagents can be washed away, facilitating easy isolation of the growing polynucleotide with no need for purification between reaction steps; and the process is amenable to automation on computer-controlled solid-phase synthesisers.

Typically in solid-phase polynucleotide synthesis, a polynucleotide or an initial nucleoside/nucleotide monomer is attached to a solid support and a series of reactions are carried out to increase the chain length of the support-attached polynucleotide/initial monomer, such that synthesis is carried out on the solid support. The solid supports are typically held between filters within synthesis columns. Solution-phase reagents and solvents may pass freely through such columns while the solid support and its attached compounds are held in place. The polynucleotide or an initial nucleoside/nucleotide monomer is typically attached to the solid support using a linker.

Design of appropriate linkers for use in solid-phase polynucleotide synthesis is challenging. For example, the linker must be stable under the reaction conditions used during polynucleotide synthesis, but cleavable under specific conditions once polynucleotide synthesis is complete, in order to yield the free polynucleotide product. The linker must also be amenable to attaching to a desired position on a polynucleotide in order to facilitate controlled synthesis.

There are several known chemical linkers that have been used to attach a solid support to a polynucleotide or an initial nucleoside/nucleotide monomer for the purposes of solid-phase polynucleotide synthesis.

Most commonly, a succinyl-based linker is used. An example of a succinyl-based linker is shown below.

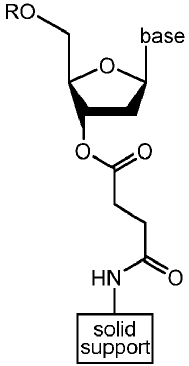

Succinyl linkers can be cleaved by hydrolysis to release the free nucleotide without any further necessary reaction steps. However, the reaction conditions for succinyl cleavage are relatively harsh and typically require the use of concentrated ammonium hydroxide. The use of harsh reaction conditions increases the complexity of equipment setup needed for the synthesis reaction and can also limit the scope of polynucleotides that can be synthesized. For example, it may not be possible to include desired modifications within the synthesized polynucleotide (e.g. specifically modified nucleotide monomers) if the chemical groups constituting such modifications are susceptible to cleavage or modification by the reagents used in the overall polymer synthesis.

Furthermore, as a practicality, when using succinyl linkers the first nucleoside/nucleotide monomer typically needs to be pre-attached to the solid support. This has the disadvantage that a different synthesis column is typically required depending on the identity of the nucleobase desired on the initial nucleotide monomer. Typically, different columns are required for each different monomer, and as such columns are typically available only for natural nucleobases (e.g. adenine (A), cytosine (C), guanine (G) or thymine (T)) and a small range of modified derivatives. However, if a non-natural nucleobase is desired a suitable column may not be readily available, which limits the range of polynucleotides that can be synthesized in such methods.

To address this issue, attempts have been made to provide linkers that can be synthesised on a solid support and then attached to any desired nucleoside/nucleotide monomer. In such 'universal supports' for polynucleotide synthesis the first monomer in the polynucleotide is typically added as the first coupling reaction, rather than being pre-attached. Universal linkers typically function by attaching the first nucleotide to be incorporated in the polynucleotide chain to a support via phosphate.

An example of a known linker for a universal support is provided below, with the first nucleotide attached:

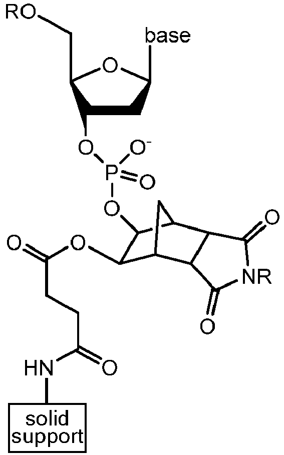

Such groups can be attached to solid supports e.g. via an amide bond as shown above with a DMT-protected OH functional group for reaction with a first nucleotide to be incorporated into the polymer chain. Once polymer synthesis is completed, ester hydrolysis releases the strained fused ring moiety of the linker following which intramolecular reactions at the phosphate group of the initial nucleotide release the polynucleotide.

Whilst such universal linkers allow a range of nucleotides to be incorporated as the initial nucleotide in a synthesized polynucleotide, significant problems remain. For example, multiple reaction steps under relatively harsh conditions are typically required in order to produce the free polynucleotide product. In particular, hydrolysis of the phosphate group typically leaves a phosphate moiety attached to the terminal sugar ring of the polynucleotide, which is typically incompatible with downstream enzymatic reactions, which usually require a terminal hydroxyl group at the corresponding position. This means that it is usually necessary to treat a synthesized polynucleotide with further reagents in order to cleave the phosphate group, adding additional steps, further cost, and the need for additional purification steps. Furthermore, if the cleaved linker is produced as a solution-phase by-product, the desired polynucleotide product must be isolated from this by-product. There is a need for linkers which address these issues.

Photocleavable or 'photolabile' linkers, which undergo cleavage under photoexcitation, are known. Photocleavable linkers have advantages in terms of relatively mild cleavage conditions that generally do not modify the structure of the polynucleotide product and which are orthogonal to typical reaction conditions used during polynucleotide synthesis.

Prior attempts have focussed on photocleavable linkers which comprise a carboxyl group to attach the initial nucleotide to the support. Such linkers have typically focussed on the use of ester or carbonate groups. Known photocleavable linkers include the following:

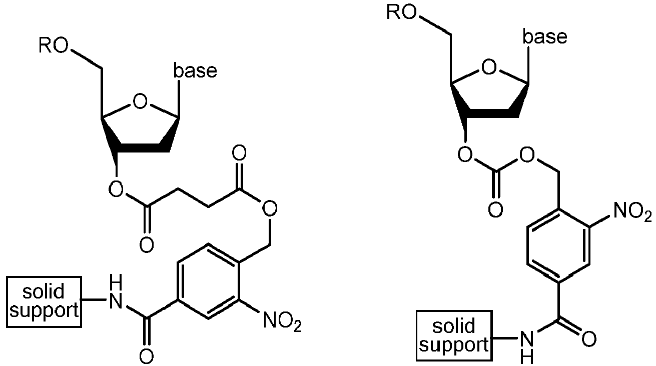

Whilst such groups can be photochemically cleaved, significant issues remain. For example, diester-comprising linkers such as the succinate-based linker above (the first of the two linkers depicted above) have the disadvantage that photocleavage of the linker typically does not produce the free nucleotide in a single reaction step, but rather produces a succinyl-functionalised nucleotide. Similarly, carbonate-comprising linkers such as the second of the two linkers depicted above produce a carbonate-functionalised nucleotide. Further reaction steps are then needed to yield the free (poly)nucleotide, which adds to the complexity, cost and time required for the synthesis procedure. Harsh reaction conditions are required to remove the succinyl or carbonate group from succinyl- or carbonate-functionalised nucleotides. As a consequence, the photocleavable linkers depicted above have similar drawbacks to the succinyl-based linkers described above, as the use of harsh reaction conditions increases the complexity of equipment setup needed for the synthesis reaction and can also limit the scope of polynucleotides that can be synthesized. For example, it may not be possible to produce (poly)nucleotides with protected nucleobases, as the nucleobase protecting groups would be at least partially removed during removal of the succinyl or carbonate group.

Furthermore, both ester and carbonate motifs used in photocleavable linkers such as those depicted above are not stable under typical hydrolysis conditions, which is problematic as explained below.

In more detail, it is common practice during polynucleotide synthesis to use protecting groups to protect various chemical groups present in the nucleosides, nucleotides and/or polynucleotides involved in the synthesis. In particular, protecting groups are often present on the nucleobases and/or phosphorous-based linkages. These protecting groups are typically removed under hydrolysis conditions.

It is often desirable to remove the protecting groups present on the nucleobases and/or the phosphodiester backbone of the synthesised polynucleotide *before* the polynucleotide is cleaved from its solid support; i.e. whilst the polynucleotide is retained on the support. This allows the by-products of the deprotection reactions, such as the removed protecting groups, to be easily washed away. However, as explained above, photocleavable linkers based on ester or carbonate bridges are not stable under typical deprotection conditions used to remove protecting groups from synthesised polynucleotides. This causes problems during the synthesis procedure. If deprotection is conducted before photocleavage then the polynucleotide product inevitably cleaves from the support, and then needs to be purified from the deprotection products, thus rendering the photocleavable nature of the linker largely superfluous. If photocleavage is conducted before the deprotection step (as necessary in practice), then downstream processing of the cleaved polynucleotide is still required and there remains a need for solution-based purification of the desired polynucleotide from cleaved protecting groups. There is thus a need for improvements in this area.

Another issue is that the saccharide ring of a nucleotide typically has multiple hydroxyl group substituents. In DNA nucleotides, there are hydroxyl groups at the 3' and 5' positions, while in RNA nucleotides there are hydroxyl groups at the 2', 3' and 5' positions. In solid-state polynucleotide synthesis, it is typically preferable for the polynucleotide/the initial monomer unit to be attached to the solid support via its 3' hydroxyl group. This is because, although polynucleotide synthesis may be performed in either the 3' to 5' direction or the 5' to 3' direction, it is often preferable to perform polynucleotide synthesis in the 3' to 5' direction. One reason that polynucleotide synthesis in the 3' to 5' direction is sometimes preferable is that the relevant monomer units for 3' to 5' direction synthesis tend to be cheaper. Another reason is that the variety of readily available monomer units is greater for 3' to 5' direction synthesis than for 5' to 3' direction synthesis. For example, a wide variety of non-natural, modified monomers suitable for 3' to 5' direction synthesis are commercially available, while this is not the case for 5' to 3' direction synthesis. A disadvantage of some known linkers is that, during synthesis of the linker between the nucleotide and the solid support, a significant amount of the linker may attach to the 2' and/or 5' hydroxyl group instead of preferentially attaching to the 3' hydroxyl group. There is likewise a need for improvements in this area.

Photocleavable groups have also been used in RNA synthesis, for example to transiently attach detectable markers to the terminal residues in synthesized strands in order to confirm strand syntheis. For example, a photocleavable linker based on an acetal bridge to a nitrobenzene group conjugated to a alkyldiamide linker has been described for selectively linking the terminal 2'-OH group of an RNA polymer to biotin, for attachment to a streptavidin-functionalised bead in order to confirm successful strand synthesis.

The use of chemical linkers to attach a first (poly)nucleotide to a second (poly)nucleotide is also known. When a linker is attached to the 3' end of one (poly)nucleotide and the 5' end of the other (poly)nucleotide, the overall structure can be considered as single polynucleotide strand with an interrupting linker unit. When the interrupting linker unit is photolabile, it provides a means of cleaving the polynucleotide strand into two separate (poly)nucleotide components *in situ* using photoexcitation. Such linkers are referred to as 'caged strand breakers'.

An example of a known caged strand breaker is 'caged strand breaker II CEP'. The structure of 'caged strand breaker II CEP' in its unattached form is provided below:

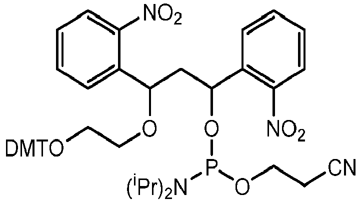

When this linker is introduced to a polynucleotide sequence during polynucleotide synthesis, it is possible to produce a polynucleotide strand with a photolabile interrupting linker having the structure provided below:

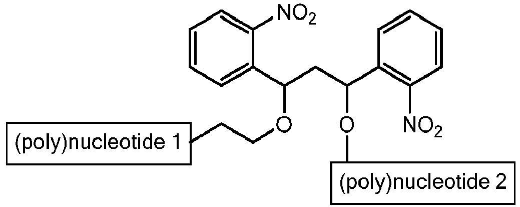

Whilst linkers such as 'caged strand breaker II CEP' enable the *in-situ* photochemical cleavage of polynucleotide strands, significant issues remain.

For example, it may be impractical to use a phosphoramidite nucleotide monomer to introduce the first 3' nucleotide to 'caged strand breaker II CEP'. This is because the resulting phosphate diester bond is anionic and is therefore less reactive to elimination during the photocleavage of the 'caged strand breaker II CEP' linker. It may therefore be necessary to introduce the first 3' nucleotide using a methyl nucleotide phosphonamidite monomer, which results in a neutrally charged methyl phosphonate bond.

Phosphoramidite monomers are significantly less expensive than methyl phosphonamidite monomers, and a much wider range of phosphoramidite monomers are commercially available, including non-natural and modified nucleotide phosphoramidite monomers. It is therefore desirable to be able to use a phosphoramidite monomer to introduce a first 3' nucleotide to a linker, and there is a need for improvements in this area.

Thus, it is desirable to provide a linker for attaching polynucleotides or their monomer units to a solid support that: (i) may be cleaved under mild conditions that are orthogonal to reaction conditions typically used in polynucleotide synthesis; (ii) cleaves in a single reaction step to provide the free polynucleotide with a minimum of reaction by-products; (iii) is stable under reaction conditions typically used to deprotect synthesised polynucleotides; and (iv) preferentially attaches to the 3' hydroxyl group of a polynucleotide/monomer unit.

It is also desirable to provide a linker for attaching a first polynucleotide to a second polynucleotide that is compatible with phosphoramidite monomers.

The present invention aims to address some or all of these issues.

### Summary

The inventors have developed compounds of formula (I) as excellent photocleavable nucleosides, nucleotides, polynucleotides and derivatives and analogues thereof suitable for use in solid-phase polynucleotide synthesis. In particular, the linker present in these compounds typically has each of the desired advantages (i) to (iv) set out above.

The present invention therefore provides a method of producing a polynucleotide or an analogue or derivative thereof, comprising:
(i) providing a support-attached nucleoside, nucleotide, polynucleotide or derivative or analogue thereof, which is a compound of formula (I) attached to a solid support at Ⓐ, 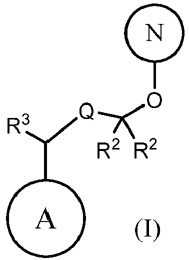 wherein:
   - Ⓝ is a nucleoside, nucleotide, polynucleotide or derivative or analogue thereof,
   - Q is an oxygen atom or a sulphur atom,
   - each R² is independently selected from hydrogen, methyl, ethyl, C₁ to C₂ haloalkyl and a halogen group,
   - R³ is methyl, ethyl or C₁ to C₂ haloalkyl, and
   - Ⓐ is a 2-nitrobenzyl group, wherein said 2-nitrobenzyl group is optionally substituted with 1, 2 or 3 groups independently selected from halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, wherein each R^{a} is independently selected from hydrogen, optionally substituted C₁ to C₂ alkyl and optionally substituted C₁ to C₂ alkoxyl and each R^{b} is independently selected from hydrogen and an
      optionally substituted C₁ to C₄ alkyl group;
(ii) increasing the chain length of the support-attached nucleoside, nucleotide, polynucleotide or derivative or analogue thereof by introducing one or more additional nucleoside units, nucleotide units and/or analogues or derivatives thereof; and
(iii) cleaving the resulting polynucleotide from the solid support by photo-illuminating the compound of formula (I).

In some embodiments, Q is an oxygen atom, and/or each R² is independently selected from hydrogen, methyl, C₁ fluoroalkyl and fluorine; preferably each R² is hydrogen; and/or R³ is methyl, ethyl or C₁ to C₂ fluoroalkyl; preferably R³ is methyl.

In some embodiments Ⓐ is covalently attached to a linking group and the linking group is covalently attached to the solid support.

In some embodiments Ⓐ is represented by formula (A-1) or formula (A-2), 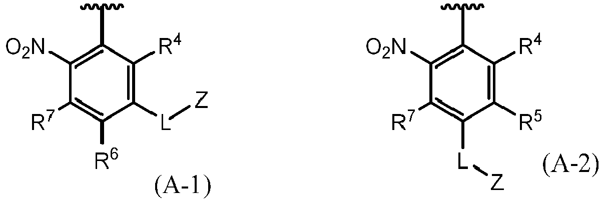 wherein R⁴, R⁵, R⁶ and R⁷ are each independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, wherein R^{a} and R^{b} are as defined herein; L is the linking group; and Z is the solid support.

In some embodiments, R⁴, R⁵, R⁶ and R⁷ are each independently selected from hydrogen, fluorine, optionally substituted C₁ to C₄ alkyl, -OR^{a}, -SR^{a}, -NR^{a}R^{a}, -OC(O)R^{b} and -NHC(O)R^{b}, wherein R^{a} and R^{b} are as defined herein; wherein preferably R⁴, R⁵, R⁶ and R⁷ are each hydrogen or methoxy.

In some embodiments, the linking group comprises a C₁ to C₂₀ alkylene group, a C₂ to C₂₀ alkenylene group, a C₂ to C₂₀ alkynylene group and/or a nucleotide or a polynucleotide, wherein said alkylene, alkenylene or alkynylene group is optionally interrupted by and/or terminated in one or more groups selected from: a heteroatom; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered saturated or partially unsaturated heterocyclic group; and wherein the linking group is optionally further substituted. In some embodiments, the linking group is attached to the solid support via a phosphoramidite group.

In some embodiments, the solid support comprises particles having a diameter of from about 10 to about 1000 µm.

In some embodiments, the solid support comprises a glass, silica, ceramic, or a polymeric resin.

In some embodiments, Ⓝ is connected to the adjacent oxygen atom of formula (I) at the 3' position or the 5' position of the nucleoside, nucleotide, polynucleotide or derivative or analogue thereof.

In some embodiments, Ⓝ is represented by formula (N-1) or formula (N-2), 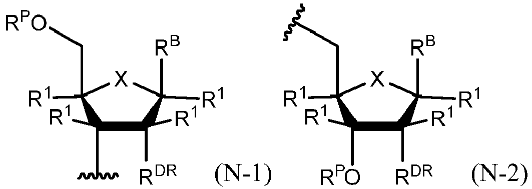 wherein X is an oxygen atom, a nitrogen atom, a sulphur atom or -C(R^{a}R^{a})-; each R¹ and R^{DR} is independently selected from hydrogen, halogen, optionally substituted C₁ to C4 alkyl, -OR^{a}, -SR^{a}, -NR^{a}R^{a}, -C(O)OR^{a}, -C(O)NR^{a}R^{a} and -C(O)R^{b}, wherein R^{a} and R^{b} are as defined herein, and when R^{DR} is -OR^{a} the R^{a} group and one of the R¹ groups may be joined together to form a bridging motif; R^{P} is a hydrogen, hydroxyl protecting group, phosphoryl group or a salt or acid therof, a diphosphoryl group (-P(O₂⁻)-O-P(O₃⁻)) or a salt or acid thereof, a triphosphoryl group (-P(O₂⁻)-P(O₂⁻)-O-P(O₃⁻)) or a salt or acid thereof, or a phosphorous-based linkage to a nucleoside, nucleotide or polynucleotide or a derivative or analogue thereof; R^{B} is an optionally substituted natural or non-natural nucleobase or a derivative or analogue thereof, and the wavy line indicates the point of attachment to the compound of formula (I).

In some embodiments, X is an oxygen atom; and/or each R¹ is independently selected from hydrogen, halogen, C₁ to C₂ alkyl, C₁ to C₂ haloalkyl and C₁ to C₂ alkoxyl; preferably each R¹ is hydrogen; and/or R^{DR} is a hydrogen or a halogen; preferably R^{DR} is hydrogen and/or R^{P} is a hydrogen or a hydroxyl protecting group.

In some embodiments, step (i) comprises:
(a) providing a compound of formula (Ia), 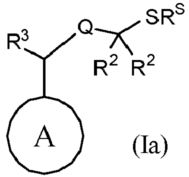 wherein Q, R², R³ and Ⓐ are as defined herein, and R^{S} is a C₁ to C₄ alkyl; and
(b) reacting the compound of formula (Ia) with a nucleoside, nucleotide, polynucleotide or derivative or analogue thereof; wherein preferably the nucleoside, nucleotide, polynucleotide or derivative or analogue thereof is represented by formula (N-1) or formula (N-2) as defined herein;
   thereby forming a compound of formula (I).

In some embodiments, step (i) comprises attaching a compound of formula (I) or formula (Ia) to a solid support, 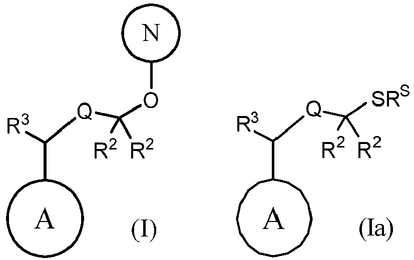 wherein Ⓝ, Q, R², R³, R^{S} and Ⓐ are as defined herein.

In some embodiments, increasing the chain length in step (ii) comprises performing the following step one or more times: contacting the support-attached nucleoside, nucleotide, polynucleotide, or derivative or analogue thereof with a nucleoside or a derivative thereof, thereby forming a phosphorous-based linkage.

In some embodiments, increasing the chain length in step (ii) comprises performing the following steps (a) to (e) one or more times:
(a) providing a nucleoside having: (i) a phosphoramidite group at the 3' position and a hydroxyl protecting group at the 5' position; or (ii) a phosporamidite group at the 5' position and a hydroxyl protecting group at the 3' position;
(b) activating the nucleoside phosphoramidite of step (a);
(c) contacting the support-attached nucleoside, nucleotide, polynucleotide, or derivative or analogue thereof with the activated nucleoside phosphoramidite, thereby forming a phosphite triester linkage;
(d) oxidising the phosphite triester linkage to form a phosphotriester linkage; and
(e) removing the hydroxyl protecting group from the terminal nucleoside of the resulting support-attached polynucleotide.

In some embodiments, after step (ii) and before step (iii), the method further comprises one or more steps of deprotecting the support-attached polynucleotide.

In some embodiments, step (iii) comprises photo-illuminating the compound of formula (I) using UV light at a wavelength of about 300 to about 500 nm.

Also provided herein is a support-attached nucleoside, nucleotide, or derivative or analogue thereof, which is a compound of formula (I) attached to a solid support at Ⓐ, 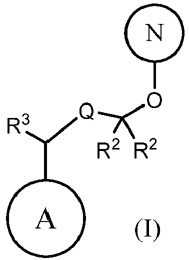 wherein Q, R², R³ and Ⓐ are as defined herein; Ⓝ is a nucleoside, nucleotide or derivative or analogue thereof, wherein preferably the nucleoside, nucleotide, or derivative or analogue thereof is represented by formula (N-1) or formula (N-2) as defined herein.

Also provided herein is a compound of formula (I), 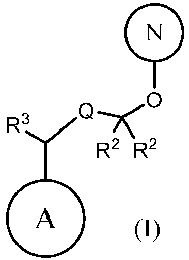 wherein Q, R², R³ and Ⓐ are as defined herein, Ⓝ is represented by formula (N-1) or formula (N-2) as defined herein, and Ⓐ may be optionally attached to a solid support. Accordingly, in such compounds, the solid support may be present or absent.

Also provided herein is a support-attached compound of formula (Ia), which is attached to a solid support at Ⓐ, 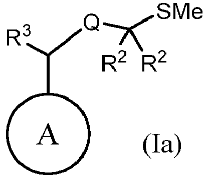 wherein Q, R², R³ and Ⓐ are as defined herein.

Also provided herein is a method of producing a support-attached nucleoside, nucleotide or derivative or analogue thereof as defined herein, comprising: (a) providing a compound of formula (Ia) which is attached to a solid support at Ⓐ as defined herein; and (b) reacting the compound of formula (Ia) with a nucleoside, nucleotide or derivative or analogue thereof to produce a support-attached nucleoside, nucleotide or derivative or analogue thereof.

Also provided herein is a solid support column for solid-phase polynucleotide synthesis, comprising a support-attached nucleoside, nucleotide, polynucleotide or a derivative or analogue thereof which is a compound of formula (I) attached to a solid support at Ⓐ, 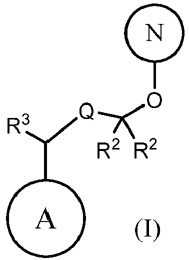 wherein Ⓝ, Q, R², R³ and Ⓐ are as defined herein.

Also provided is use of a support-attached nucleoside, nucleotide or derivative or analogue thereof as defined herein, a compound of formula (I) as defined herein, or a compound of formula (Ia) as defined herein, for synthesising polynucleotides or derivatives or analogues thereof.

Further provided is a polynucleotide or derivative or analogue thereof, obtainable by a method as defined herein.

### Detailed Description of the Invention

### Definitions

The term "nucleosides, nucleotides, polynucleotides and derivatives and analogues thereof' includes: nucleosides; derivatives of nucleosides; analogues of nucleosides; nucleotides; derivatives of nucleotides; analogues of nucleotides; polynucleotides; derivatives of polynucleotides; and analogues of polynucleotides.

As used herein, "derivatives" of nucleosides, nucleotides or polynucleotides are modified forms of nucleosides, nucleotides or polynucleotides which comprise one or more chemical modifications to the phosphate backbone, sugar ring or nucleobase of the DNA or RNA. A derivative of a nucleoside thus may be a chemically modified nucleoside as described in more detail herein.

As used herein "analogs" of nucleosides, nucleotides or polynucleotides are compounds which are structurally similar to nucleotides such as DNA and RNA but which include modifications at one or more positions such as at the phosphate backbone, sugar ring or nucleobase of the DNA or RNA. Nucleotide analogs include peptide nucleic acid (PNA), glycerol nucleic acid (GNA), threose nucleic acid (TNA), locked nucleic acid (LNA) and other synthetic polymers with nucleotide side chains.

Unless otherwise specified, the term "nucleoside" includes protected nucleosides - i.e. nucleosides that have been modified to include protecting groups in their chemical structure. Unless otherwise specified, the term "nucleotide" includes protected nucleotides - i.e. nucleotides that have been modified to include protecting groups in their chemical structure. Unless otherwise specified, the term "polynucleotide" includes protected polynucleotides - i.e. polynucleotides that have been modified to include protecting groups in their chemical structure. Such protecting groups are typically distinct from modifications that may be made in nucleotide derivatives for functional reasons e.g. to alter the properties of the nucleotide or polynucleotide.

As used herein and unless otherwise specified, the term "protecting group" refers to any suitable protecting group known in the field. Different protecting groups are suitable for protecting different chemical groups. For example, as used herein and unless otherwise specified, the term "hydroxyl protecting group" refers to any protecting group suitable for protecting hydroxyl groups that is known in the field. Similarly, as used herein and unless otherwise specified, the term "amine protecting group" refers to any protecting group suitable for protecting amine groups that is known in the field. Furthermore, as used herein and unless otherwise specified, the terms "phosphate protecting group" and "phosphite protecting group" refer to any protecting group suitable for protecting a phosphate or phosphite group, respectively, that is known in the field.

Suitable hydroxyl protecting groups include acetyl (Ac), benzoyl (Bz), benzyl (Bn), β-methoxyethoxymethyl ether (MEM), 4,4'-dimethoxytrityl (DMT), methoxymethyl ether (MOM), 4-monomethoxytrityl (MMT), levulinyl, 9-fluorenylmethyloxycarbonyl (Fmoc) *p*-methoxybenzyl ether (PMB), *p*-methoxyphenyl ether (PMP), methylthiomethyl ether, pivaloyl (Piv), tetrahydropyranyl (THP), tetrahydrofuran (THF), trityl (Tr), silyl ethers such as trimethylsilyl (TMS), *tert*-butyldimethylsilyl (TBDMS), tri-*iso-*propylsilyloxymethyl (TOM) and triisopropylsilyl (TIPS), methyl ether, ethoxyethyl ether, allyl ether and tert-butyl ether.

Suitable amine protecting groups include carbobenzyloxy (Cbz), *p*-methoxybenzyl carbonyl (Moz), *tert*-butyloxycarbonyl (BOC), 9-fluorenylmethyloxycarbonyl (Fmoc), acetyl (Ac), trifluoroacetyl, benzoyl (Bz), benzyl (Bn), benzylidene, trityl (Tr), carbamate, phthalimide, *p*-methoxybenzyl (PMB), 3,4-dimethoxybenzyl (DMPM), p-methoxyphenyl (PMP), trichloroethyl chloroformate (Troc), tosyl (Ts), nosyl and 2-nitrophenylsulfenyl (Nps).

Suitable phosphate protecting groups and phosphite protecting groups include: alkyl groups, such as C₁ to C₄ alkyl groups optionally substituted with 1, 2 or 3 groups independently selected from halogen, -OR^{a}, -SR^{a}, -NR^{a}R^{a}, -C(O)OR^{a}, -C(O)NR^{a}R^{a}, -C(O)R^{b}, -OC(O)R^{b}, -NHC(O)R^{b} and -CN, and in particular methyl, ethyl and 2-cyanoethyl, preferably 2-cyanoethyl; o-chlorophenyl; and p-chlorophenyl.

As used herein, an alkyl group can be linear, branched or cyclic but is preferably linear. A C₁ to C₂₀ alkyl group is a linear or branched alkyl group containing from 1 to 20 carbon atoms. A C₁ to C₂₀ alkyl group is typically a C₁ to C₁₀ alkyl group such as a C₁ to C₆ alkyl group, a C₁ to C₅ alkyl group or a C₁ to C₄ alkyl group. Suitable C₁ to C₄ alkyl groups include methyl, ethyl, n-propyl, i-propyl, n-butyl, sec-butyl and tert-butyl. A C₁ to C₄ alkyl group is preferably a C₁ to C₃ alkyl group, more preferably ethyl or methyl. Unless otherwise specified, an alkyl group can be unsubstituted or substituted as described herein. For the avoidance of doubt, where two alkyl groups are present, the alkyl groups may be the same or different.

As used herein, an alkenyl group can be linear, branched or cyclic but is preferably linear. An alkenyl group has one or more, e.g. one or two, typically one double bonds. A C₂ to C₂₀ alkenyl group is a linear or branched alkenyl group containing from 2 to 20 carbon atoms. A C₂ to C₂₀ alkenyl group is typically a C₂ to C₁₀ alkenyl group such as a C₂ to C₆ alkenyl group, a C₂ to C₅ alkenyl group or a C₂ to C₄ alkenyl group. A C₂ to C₄ alkenyl group is preferably a C₂ to C₃ alkenyl group. Examples of C₂-C₄ alkenyl groups include ethenyl, propenyl and butenyl. Unless otherwise specified, an alkenyl group can be unsubstituted or substituted as described herein. For the avoidance of doubt, where two alkenyl groups are present, the alkenyl groups may be the same or different.

As used herein, an alkynyl group can be linear, branched or cyclic but is preferably linear. An alkenyl group has one or more, e.g. one or two, typically one triple bonds. A C₂ to C₂₀ alkynyl group is a linear or branched alkynyl group containing from 2 to 20 carbon atoms. A C₂ to C₂₀ alkynyl group is typically a C₂ to C₁₀ alkynyl group such as a C₂ to C₆ alkynyl group, a C₂ to C₅ alkynyl group or a C₂ to C₄ alkynyl group. A C₂ to C₄ alkynyl group is preferably a C₂ to C₃ alkynyl group. Examples of C₂ to C₄ alkynyl groups include ethynyl, propynyl and butynyl. Unless otherwise specified, an alkynyl group can be unsubstituted or substituted as described herein. For the avoidance of doubt, where two alkynyl groups are present, the alkynyl groups may be the same or different.

As used herein, an alkylene group is a an unsubstituted or substituted bidentate moiety obtained by removing two hydrogen atoms from an alkane. The two hydrogen atoms may be removed from the same carbon atom or from different carbon atoms. As used herein, an alkylene group can be linear, branched or cyclic but is preferably linear. A C₁ to C₂₀ alkylene group is a linear or branched alkylene group containing from 1 to 20 carbon atoms. A C₁ to C₂₀ alkylene group is typically a C₁ to C₁₀ alkylene group such as a C₁ to C₆ alkylene group, a C₁ to C₅ alkylene group or a C₁ to C₄ alkylene group. Suitable C₁ to C₄ alkylene groups include methylene, ethylene, n-propylene, i-propylene, n-butylene, sec-butylene and tert-butylene. A C₁ to C₄ alkylene group is preferably a C₁ to C₃ alkylene group, more preferably ethylene or methylene. Unless otherwise specified, an alkylene group can be unsubstituted or substituted as described herein. For the avoidance of doubt, where two alkylene groups are present, the alkylene groups may be the same or different.

As used herein, an alkenylene group is a an unsubstituted or substituted bidentate moiety obtained by removing two hydrogen atoms from an alkene. The two hydrogen atoms may be removed from the same carbon atom or from different carbon atoms. As used herein, an alkenylene group can be linear, branched or cyclic but is preferably linear. An alkenylene group has one or more, e.g. one or two, typically one double bonds. A C₂ to C₂₀ alkenylene group is a linear or branched alkenylene group containing from 2 to 20 carbon atoms. A C₂ to C₂₀ alkenylene group is typically a C₂ to C₁₀ alkenylene group such as a C₂ to C₆ alkenylene group, a C₂ to C₅ alkenylene group or a C₂ to C₄ alkenylene group. A C₂ to C₄ alkenylene group is preferably a C₂ to C₃ alkenylene group. Examples of C₂-C₄ alkenylene groups include ethenylene, propenylene and butenylene. Unless otherwise specified, an alkenylene group can be unsubstituted or substituted as described herein. For the avoidance of doubt, where two alkenylene groups are present, the alkenylene groups may be the same or different.

As used herein, an alkynylene group is a an unsubstituted or substituted bidentate moiety obtained by removing two hydrogen atoms from an alkyne. As used herein, an alkynylene group can be linear, branched or cyclic but is preferably linear. An alkynylene group has one or more, e.g. one or two, typically one triple bonds. A C₂ to C₂₀ alkynylene group is a linear or branched alkynylene group containing from 2 to 20 carbon atoms. A C₂ to C₂₀ alkynylene group is typically a C₂ to C₁₀ alkynylene group such as a C₂ to C₆ alkynylene group, a C₂ to C₅ alkynylene group or a C₂ to C₄ alkynylene group. A C₂ to C₄ alkynylene group is preferably a C₂ to C₃ alkynylene group. Examples of C₂-C₄ alkynylene groups include ethynylene, propynylene and butynylene. Unless otherwise specified, an alkynylene group can be unsubstituted or substituted as described herein. For the avoidance of doubt, where two alkynylene groups are present, the alkynylene groups may be the same or different.

As used herein, a halogen is typically chlorine, fluorine, bromine or iodine, and is preferably chlorine, fluorine or bromine, more preferably chlorine or fluorine.

As used herein, a C₆ to C₁₀ aryl group is an aryl group having from 6 to 10 carbon atoms, for example, phenyl or naphthyl, preferably phenyl. An aryl group or moiety can be substituted or unsubstituted as described herein.

As used herein, a C₅ to C₁₀ carbocyclyl group can be a C₅, C₆, C₇, C₈, C₉ or C₁₀ cycloalkyl group and is preferably cyclopentyl or cyclohexyl. Typically a cycloalkyl group is substituted or unsubstituted with up to three substituents, e.g. one or two substituents.

As used herein, a 5- to 10-membered heteroaryl group is a 5- to 10-membered ring system in which the ring is fully unsaturated and aromatic and contains at least one heteroatom. Typically, the ring contains up to three or four heteroatoms, e.g. one or two heteroatoms, selected from O, N and S. Thus, a 5- to 10-membered heteroaryl group is typically a 5- to 10-membered aromatic ring containing one, two or three heteroatoms selected from O, N and S. Preferably, the heteroatoms are selected from O and N. Suitable such heteroaryl groups include, for example:
monocyclic 5- to 7-membered heteroaryl rings such as furanyl, oxepinyl, pyrrolyl, pyrazolyl, imidazolyl, triazolyl, tetrazolyl, oxazolyl, isoxazolyl, oxadiazolyl, thiazolyl, isothiazolyl, thiadiazolyl, pyridinyl, pyradazinyl, pyrimidinyl, pyrazinyl, triazinyl, azepinyl, thiophenyl, oxepinyl and thiepinyl; and
bicyclic 8- to 10-membered heteroaryl rings such as benzofuranyl, indolyl, isoindolyl, indolizinyl, indazolyl, benzimidazolyl, azaindolyl, azaindazolyl, purinyl, benzooxazolyl, benzoisooxazolyl, benzothiazolyl, benzoisothiazolyl, benzothiadiazolyl, quinolinyl, isoquinolinyl, quinolizinyl, quinoxalinyl, phthalazinyl, quinazolinyl, cinnolinyl, naphthyridinyl, pteridinyl and benzothiophenyl, preferably benzofuranyl, indolyl, isoindolyl, quinolinyl and isoquinolinyl.

Preferably, the 5- to 10-membered heteroaryl group is a monocyclic 5- to 7-membered heteroaryl ring selected from furanyl, pyrrolyl, pyrazolyl, imidazolyl, triazolyl, oxazolyl, isoxazolyl, pyridinyl, pyradazinyl, pyrimidinyl and pyrazinyl.

As used herein, a 5- to 10-membered saturated heterocyclic group is a saturated 5-to 10-membered ring system in which the ring contains at least one heteroatom. Typically, the ring contains up to three or four heteroatoms, e.g. one or two heteroatoms, selected from O, S and N. Thus, a 5- to 10-membered saturated heterocyclic group is typically a 5-to 10-membered ring containing one, two or three heteroatoms selected from O, S and N. Suitable such saturated heterocyclic groups include, for example, monocyclic saturated 5-to 8-membered rings, more preferably 5- to 7-membered rings, such as tetrahydrofuranyl, piperidinyl, oxazolidinyl, morpholinyl, thiomorpholinyl, pyrrolidinyl, dioxolanyl, piperidonyl, azepanyl, oxepanyl, piperazinyl, tetrahydropyranyl and 1,4-diazepanyl, more preferably pyrrolidinyl, morpholinyl, piperazinyl, tetrahydropyranyl, piperidinyl, azepanyl and 1,4-diazepanyl.

As used herein, a 5- to 10-membered partially unsaturated heterocyclic group is a 5- to 10-membered ring system in which the ring contains at least one unsaturated bond and at least one heteroatom, but the ring is not fully unsaturated or aromatic. Typically, the ring contains up to three or four heteroatoms, e.g. one or two heteroatoms, selected from O, N and S. Thus, a 5- to 10-membered partially unsaturated heterocyclic group is typically a 5- to 10-membered ring containing one, two or three heteroatoms selected from O, N and S. Preferably, the heteroatoms are selected from O and N. Suitable such partially unsaturated heterocyclic groups include, for example:
monocyclic partially unsaturated 5- to 7-membered heterocyclic rings such as dihydrofuranyl, pyranyl, dihydropyranyl, dioxinyl, dihydrooxepinyl, tetrahydrooxepinyl, pyrrolinyl, pyrazolinyl, imidazolinyl, dihydrooxazolyl, dihydroisoxazolyl, dihydrothiazolyl, dihydroisothiazolyl, dihydropyridinyl, tetrahydropyridinyl, dihydropyridazinyl, tetrahydropyridazinyl, dihydropyrimidinyl, tetrahydropyrimidinyl, dihydropyrazinyl, tetrahydropyrazinyl, oxazinyl, dihydrooxazinyl, thiazinyl, dihydrothiazinyl, dihydroazepinyl, tetrahydroazepinyl, dihydrothiophenyl, thiopyranyl, dihydrothiopyranyl, dihydrothiepinyl, and tetrahydrothiepinyl; and
bicyclic partially unsaturated 8- to 10-membered heterocyclic rings such as dihydrobenzofuranyl, dihydroisobenzofuranyl, benzopyranyl, dihydrobenzopyranyl, benzodioxolyl, indolinyl, isoindolinyl, dihydroquinolinyl, tetrahydroquinolinyl, benzooxazinyl, dihydrobenzothiophenyl and benzodithiole; preferably dihydrobenzofuranyl, benzopyranyl, dihydrobenzopyranyl, benzodioxolyl, indolinyl, isoindolinyl, dihydroquinolinyl and tetrahydroquinolinyl.

Preferably, the 5- to 10-membered partially unsaturated heterocyclic group is a monocyclic partially unsaturated 5- to 7-membered ring selected from dihydrofuranyl, pyranyl, pyrrolinyl and oxazinyl.

A heterocyclic and/or heteroaryl group may be substituted or unsubstituted. Each ring atom may be unsubstituted or may carry one or two substituents. A nitrogen atom may be disubstituted, providing a positively charged heteroatom. A sulphur atom may be substituted, providing a positively charged heteroatom. Typically, a heterocyclic or heteroaryl group has up to three substituents, e.g. one or two substituents. The heterocyclic or heteroaryl ring may be connected to the remainder of the molecule by a bond to any of the available ring positions on the heterocyclic or heteroaryl ring.

As used herein, a group which is optionally substituted may unless otherwise specified be substituted with suitable substituents which may include a halogen such as chlorine and/or fluorine, a cyano group, -OR^{x}, -SR^{x}, -NR^{x}R^{x}, -C(O)OR^{x}, -C(O)NR^{x}R^{x},-C(O)R^{x} and a C₁ to C₄ alkyl group such as methyl and/or ethyl, wherein a C₁ to C₄ alkyl substituent is itself either unsubstituted or substituted with 1 to 3 halogen atoms. Each R^{x} is independently selected from hydrogen and a C₁ to C₄ alkyl group which is unsubstituted or is substituted with 1, 2 or 3 halogen groups. An optional substituent is preferably a hydroxyl group, a halogen such as chlorine or fluorine, or a C₁ to C₄ alkyl group such as methyl or ethyl.

Unless otherwise specified, included in the above are the well known ionic, salt, solvate, and protected forms of these substituents. For example, a reference to carboxylic acid, carboxy or carboxyl group (-COOH) also includes the anionic (carboxylate) form (-COO⁻), a salt or solvate thereof, as well as conventional protected forms. Similarly, a reference to an amino group includes the protonated form (-N⁺HR¹R²), a salt or solvate of the amino group, for example, a hydrochloride salt, as well as conventional protected forms of an amino group. Similarly, a reference to a hydroxy or hydroxyl group (-OH) also includes the anionic form (-O⁻), a salt or solvate thereof, as well as conventional protected forms. Salts include salts formed with acids and bases. Suitable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic or *p*-toluenesulphonic acid. Suitable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts. When intended for use in pharmaceutical applications, salts of the compounds provided herein may be preferably pharmaceutically acceptable salts.

Certain compounds may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and l-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms"). Unless otherwise specified, a reference to a particular compound includes all such isomeric forms, including (wholly or partially) racemic and other mixtures thereof. Methods for the preparation (e.g., asymmetric synthesis) and separation (e.g., fractional crystallisation and chromatographic means) of such isomeric forms are either known in the art or are readily obtained by adapting known methods, in a known manner.

Unless otherwise specified, a reference to a particular compound or complex also includes ionic, salt, solvated and protected forms.

### The photocleavable linker

In some embodiments the present invention provides a method for producing polynucleotides. The method comprises providing a support-attached nucleoside, nucleotide, polynucleotide or derivative or analog thereof which is a compound of formula (I) as defined herein, attached to a solid support as described herein at group Ⓐ of formula (I). 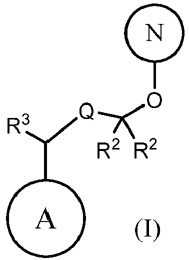 wherein Ⓝ, Ⓐ, Q, R² and R³ are as defined above and below.

Also provided are compounds of formula (I) per se as well as derivatives thereof such as compounds of formula (Ia) as described herein. The compounds of formula (I) have utility in preparing support-attached compounds as used in the provided methods and have other applications also as described herein.

The compound of formula (I) is a nucleoside, nucleotide, polynucleotide or derivative or analogue thereof attached to a photocleavable linker. In formula (I) and related formulae provided herein, Ⓝ is the nucleoside, nucleotide, polynucleotide or derivative or analogue thereof, and the remainder of formula (I) or the related formula forms a photocleavable linker that cleaves upon sufficient photoexcitation. Photocleavage is described in more detail herein. When this photocleavable linker is cleaved using photoexcitation, Ⓝ is cleaved from the photocleavable linker such that a direct product of the cleavage is a nucleoside, nucleotide, polynucleotide or derivative or analogue thereof having a hydroxyl group in place of the photocleavable linker. Accordingly, using photoexcitation, a compound of formula (I) can be converted to a compound represented by Ⓝ-OH in a single reaction step.

In more detail, Ⓐ is a 2-nitrobenzyl group that facilitates cleavage of the linker during photoexcitation. Without being bound by theory, the inventors believe that the mechanism of this cleavage is as shown by way of one specific example, below. 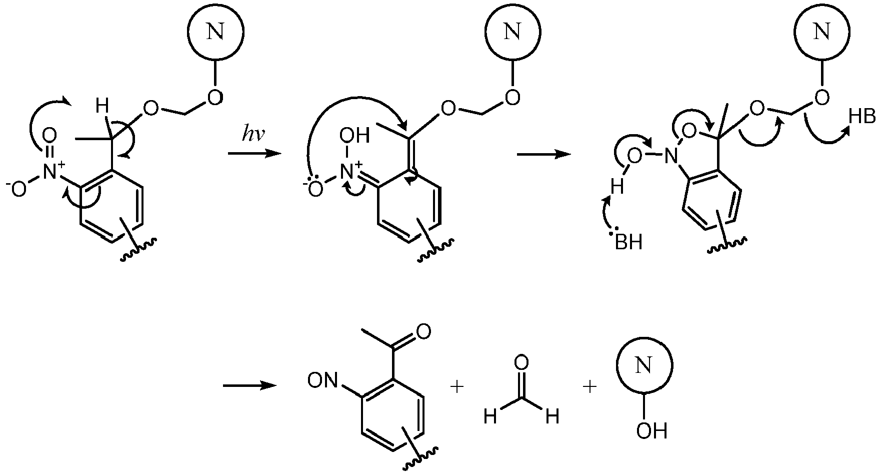 wherein HB is an acid and B is a base. However, the present invention is not limited to this specific compound or to methods using this specific compound.

Those skilled in the art will appreciate that the compound of formula (I) advantageously contains a photocleavable linker that can be cleaved using photoexcitation under mild conditions that are orthogonal to reaction conditions typically used in polynucleotide synthesis. This photocleavable linker advantageously cleaves in a single, typically concerted reaction step to provide the free nucleoside, nucleotide, polynucleotide or derivative or analogue thereof. Furthermore, the ketone by-product is volatile and thus easily removed.

The photocleavable linker present in the compound of formula (I) does not include a hydrolysable motif such as an ester or carbonate group. Accordingly, it is typically stable under reaction conditions typically used to deprotect synthesised polynucleotides - for example, deprotecting conditions used to remove hydrolysable protecting groups from protected nucleobases present in synthesised polynucleotides. For example, typically the photocleavable linker is stable with respect to concentrated ammonium hydroxide and/or ammonia. This advantageously allows various protecting groups to be removed from a synthesised polynucleotide before it is cleaved from its solid support, enabling facile isolation of the deprotected polynucleotide.

The present inventors have also found that, during the synthesis of the photocleavable linker present in the compound of formula (I), the linker advantageously preferentially attaches to the nucleoside, nucleotide, polynucleotide or derivative or analogue thereof at the 3' hydroxyl group of said nucleoside, nucleotide, polynucleotide or derivative or analogue thereof. For example, when contacted with a (poly)nucleotide having a free 3'-OH and a free 5'-OH group the linker typically bonds more readily to the 3' hydroxyl group than to the 5'-OH group. When contacted with a (poly)nucleotide having a free 2'-OH group, a free 3'-OH and a free 5'-OH group the linker typically bonds more readily to the 3' hydroxyl group than to the 2'-OH or 5'-OH group. Preferably, in the invention, the linker does not bond to a 2'-hydroxyl group. Thus, it is relatively easy to produce a compound of formula (I) in which the nucleoside, nucleotide, polynucleotide or derivative or analogue thereof is attached to the adjacent oxygen atom at the 3' position. Such compounds are particularly useful for performing polynucleotide synthesis in the 3' to 5' direction. However, those skilled in the art will appreciate that the linker can still usefully bond to e.g. a 5'-hydroxyl group if required: this is an operational parameter of the invention. For example, when contacted with a (poly)nucleotide having a free 5'-OH but no free 3'-OH group the linker will bonds to the 5' hydroxyl group.

The methods and compounds of the present invention are described in more detail below.

### Formula (I)

The present invention provides methods comprising the use of a compound of formula (I). The present invention also provides compounds of formula (I) *per se.* In some embodiments as provided herein the compound of formula (I) is attached to a solid support at position Ⓐ. 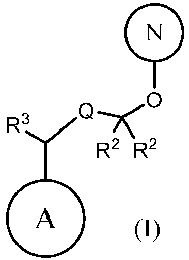 Q is an oxygen atom or a sulphur atom. Preferably, Q is an oxygen atom.

Each R² is independently selected from hydrogen, methyl, ethyl, C₁ to C₂ haloalkyl and a halogen group. Preferably, each R² is independently hydrogen, methyl, C₁ fluoroalkyl or fluorine. More preferably, each R² is independently hydrogen or methyl. More preferably, each R² is hydrogen. This can enable more facile synthesis of the compound of formula (I).

The R² groups may be the same or different. Preferably, the R² groups are the same. Preferably, one R² group is H or methyl and the other R² group is H or methyl. More preferably one R² group is H and the other R² group is H or methyl. Still more preferably both R² groups are H.

R³ is methyl, ethyl or C₁ to C₂ haloalkyl. Preferably, R³ is methyl, ethyl or C₁ to C₂ fluoroalkyl. More preferably, R³ is methyl or ethyl. Alternatively, R³ may be methyl or CF₃. Most preferably, R³ is methyl. When R³ is one of these groups, the linker that connects Ⓝ and Ⓐ may be more easily cleaved using photoexcitation and/or the linker preferentially attaches to a nucleoside at the 3' position of the sugar ring.

Ⓐ is a 2-nitrobenzyl group, wherein said 2-nitrobenzyl group is optionally substituted with 1, 2 or 3 groups independently selected from halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}.

Each R^{a} is independently selected from hydrogen, optionally substituted C₁ to C₂ alkyl and optionally substituted C₁ to C₂ alkoxyl. Preferably each R^{a} is independently selected from hydrogen or methyl, more preferably each R^{a} is hydrogen.

Each R^{b} is independently selected from hydrogen and an optionally substituted C₁ to C₄ alkyl group. Preferably each R^{b} is independently selected from hydrogen or methyl, more preferably each R^{b} is hydrogen.

As used herein, the term "2-nitrobenzyl group" refers to a nitrobenzyl group that is attached to the adjacent carbon atom of formula (I) such that the nitro group is at the 2-position relative to the -C(R³)-Q- moiety of formula (I), as indicated below.

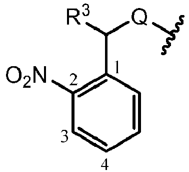

That is, in the 2-nitrobenzyl group represented by Ⓐ, the nitro group is ortho to the adjacent carbon atom of formula (I); i.e. the nitro group is ortho to the -C(R³)- carbon atom of formula (I).

The nitrobenzene group Ⓐ is, in some embodiments as provided herein, attached to a solid support. The nitrobenzene group Ⓐ may be further substituted or may be otherwise unsubstituted. When Ⓐ is further substituted it may be substituted by 1, 2 or 3 substituents independently selected from e.g. electron-donating groups. Ⓐ may be substituted by 1, 2 or 3 groups independently selected from halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and - NHC(O)R^{b}, wherein R^{a} and R^{b} are as defined above. Preferably, when Ⓐ is substituted it is substituted by 1, 2 or 3, preferably 1 or 2 groups selected from C₁ to C₄ alkyl, -OR^{a}, - SR^{a}, -NR^{a}R^{a}, -OC(O)R^{b} and -NHC(O)R^{b}. More preferably, when Ⓐ is substituted it is substituted by 1 or 2, preferably 1 group selected from OR^{a}, and -OC(O)R^{b}, preferably methoxy. Most preferably Ⓐ is unsubstituted apart from by the nitro group of the nitrobenzene and by an optional linking group attaching Ⓐ to a solid support.

In formula (I), Ⓐ is a nucleoside, nucleotide, polynucleotide or derivative or analogue thereof. Those skilled in the art will appreciate that during the method of the present invention, the chain length of the support-attached nucleoside, nucleotide, polynucleotide or derivative or analogue thereof increases. Thus, if the methods of the invention or steps thereof are repeated multiple times the composition of Ⓝ may change between cycles as the chain length increases relative to the previous step.

The nucleoside, nucleotide, polynucleotide or derivative or analogue thereof represented by Ⓝ may be selected from any suitable natural, non-natural, functionalised and/or modified nucleosides, nucleotides and polynucleotides. Such derivatives and analogues of nucleosides, nucleotides and polynucleotides are well known in the art.

For example, Ⓝ includes nucleosides, nucleotides and polynucleotides modified with protecting groups at any of their groups. In particular, it is common for such nucleosides, nucleotides and polynucleotides to have protecting groups on their saccharide rings, nucleobases and/or phosphorous-based linkages. Commonly used protecting groups are well known in the art.

For example, the amine groups present in nucleobases (such as adenine, guanine, cytosine and derivatives and analogues thereof) may be protected by amine protecting groups known in the art, which include acyl groups such as acetyl, oxyacetyl (including phenoxyacetyl and t-butylphenoxyacetyl), propionyl, isobutyryl, benzoyl and benzylidene. Formamidine groups such as dimethylformamidine and di-N-butylformamidine groups may also be used.

Phosphate linkages may be protected by phosphate protecting groups such as ethyl or 2-cyanoethyl, and phosphite linkages may be protected by phosphite protecting groups such as ethyl or 2-cyanoethyl. For example, phosphodiester backbone linkages with a protecting group may be present as phosphotriesters, for example as 2-cyanoethyl phosphotriesters.

Ⓝ also includes derivatives and analogues of nucleosides, nucleotides and polynucleotides wherein the saccharide ring is a non-natural saccharide, such as arabinose or 'locked ribose', or a saccharide ring analogue such as an iminosugar, thiosugar or carbasugar ring, in which the oxygen atom usually present in a saccharide ring is replaced with a nitrogen atom, sulphur atom or methylene motif, respectively. 'Locked ribose' is a modified ribose moiety having an extra bridge connecting the 2' oxygen and the 4' carbon. An example of a locked ribose moiety is provided below.

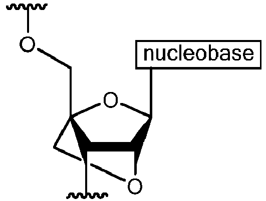

Nucleic acids containing 'locked ribose' saccharide rings are known as 'locked nucleic acids' or LNAs. LNAs have increased stability against enzymatic degradation and are a commercially important nucleic acid analogue of RNA and DNA.

Ⓝ is preferably a nucleoside, nucleotide, polynucleotide or derivative or analogue thereof wherein the saccharide rings are ribose or deoxyribose rings. Thus, Ⓝ is preferably a ribonucleoside, ribonucleotide, polyribonucleotide, deoxyribonucleoside, deoxyribonucleotide, polydeoxyribonucleotide, or a derivative or analogue thereof. More preferably, the saccharide rings are deoxyribose rings. Thus, Ⓝ is more preferably a deoxyribonucleoside, deoxyribonucleotide, polydeoxyribonucleotide, or a derivative or analogue thereof.

Ⓝ includes derivatives and analogues of polynucleotides wherein the phosphorous-based linkages connecting the nucleoside monomer units are phosphodiester linkages, phosphotriester linkages such as alkyl phosphotriester linkages, phosphite-triester linkages such as alkyl phosphotriester linkages, phosphorothioate linkages, phosphorodithioate linkages and/or alkylphosphonate linkages such as methyl phosphonate. In particular, the phosphoramidite method is a common method of polynucleotide synthesis and initially produces polynucleotides in which the phosphorous-based linkage is a phosphite-triester linkage. Such phosphite-triester linkages may be oxidised to produce phosphotriester linkages, which may be deprotected to produce phosphodiester linkages.

Ⓝ is preferably connected to the adjacent oxygen atom of formula (I) (i.e. to the - O-C(R²)₂- moiety of formula (I)) at the 3' position or the 5' position of the nucleoside, nucleotide, polynucleotide or derivative or analogue thereof represented by Ⓝ. This advantageously facilitates growth of the nucleotide chain in either the 3' to 5' direction or the 5' to 3' direction. More preferably, Ⓝ is connected to the adjacent oxygen atom of formula (I) at the 3' position. This facilitates growth of the nucleotide chain in the 3' to 5' direction, which is typically cheaper and more versatile.

Preferably, Ⓝ is a moiety represented by formula (N-1) or formula (N-2). More preferably, Ⓝ is represented by formula (N-1).

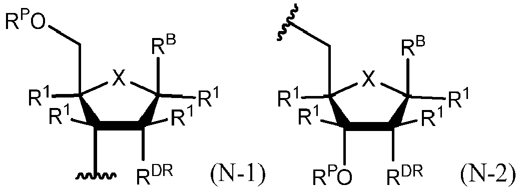

The wavy line indicates the point of attachment to the compound of formula (I); i.e. the wavy line represents a bond to the -O-C(R²)₂- moiety of formula (I)).

X is an oxygen atom, a nitrogen atom, a sulphur atom or -C(R^{a}R^{a})-. Preferably, X is an oxygen atom. When X is an oxygen atom, synthesis of the compound of formula (I) is more facile.

Each R¹ and R^{DR} is independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a} and -C(O)R^{b}, and when R^{DR} is -OR^{a} the R^{a} group and one of the R¹ groups may be joined together to form a bridging motif.

Preferably, each R¹ is independently selected from hydrogen, halogen, C₁ to C₂ alkyl, C₁ to C₂ haloalkyl and C₁ to C₂ alkoxyl. More preferably, each R¹ is selected from hydrogen, fluorine, methyl and methoxy. Further preferably, each R¹ is hydrogen.

Preferably, R^{DR} is a hydrogen, halogen, C₁ to C₂ alkyl or -OR^{a}. More preferably, R^{DR} is hydrogen, fluorine, methyl or -OR^{a}. Further preferably, R^{DR} is hydrogen, hydroxyl or methoxy. Still more preferably, R^{DR} is hydrogen or hydroxyl. Sometimes R^{DR} is not hydroxyl. Most preferably, R^{DR} is hydrogen.

R^{P} is a hydrogen, hydroxyl protecting group, phosphoryl group (-P(O₃⁻)) or a salt or acid therof, a diphosphoryl group (-P(O₂⁻)-O-P(O₃⁻)) or a salt or acid thereof, a triphosphoryl group (-P(O₂⁻)-P(O₂⁻)-O-P(O₃⁻)) or a salt or acid thereof, or a phosphorous-based linkage to a nucleoside, nucleotide or polynucleotide or a derivative or analogue thereof.

Preferably, R^{P} is a hydrogen, hydroxyl protecting group or a phosphotriester linkage to a nucleoside, nucleotide or polynucleotide or a derivative or analogue thereof. More preferably, R^{P} is a hydrogen or a hydroxyl protecting group. Further preferably, R^{P} is a hydrogen or a hydroxyl protecting group selected from a 4,4'-dimethoxytrityl group, a 4-monomethoxytrityl group, a levulinyl group and an Fmoc group. Even more preferably, R^{P} is a hydrogen or a 4,4'-dimethoxytrityl group. Most preferably, R^{P} is a 4,4'-dimethoxytrityl group.

When R^{P} is a phosphorous-based linkage to a nucleoside, nucleotide or polynucleotide or a derivative or analogue thereof, the phosphorous-based linkage may be a phosphodiester linkage, a phosphotriester linkage such as an alkyl phosphotriester linkage, a phosphite-triester linkage such as an alkyl phosphite-triester linkage, a phosphorothioate linkage, a phosphorodithioate linkage or an alkylphosphonate linkage such as methyl phosphonate. Preferably, the phosphorous-based linkage is a phosphodiester linkage, a phosphotriester linkage or a phosphite-triester linkage. Further preferably, the phosphorous-based linkage is represented by formula (P-1) or (P-2):

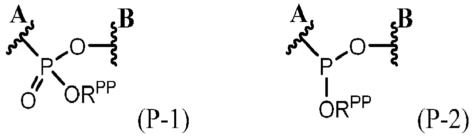

The leftmost connection point in each of formulae (P-1) and (P-2), marked as connection point A, is the connection point to the adjacent oxygen atom of formula (N-1) or (N-2). The rightmost connection point in each of in each of formulae (P-1) and (P-2), marked as connection point B, is the connection point to a nucleoside, nucleotide or polynucleotide or a derivative or analogue thereof. R^{PP} is a hydrogen, a lone pair of electrons resulting in a negative charge, a phosphate protecting group or a phosphite protecting group. Preferably, R^{PP} is a hydrogen, a lone pair of electrons resulting in a negative charge, o-chlorophenyl, p-chlorophenyl or a C₁ to C₄ alkyl group optionally substituted with 1, 2 or 3 groups independently selected from halogen, -OR^{a}, -SR^{a}, -NR^{a}R^{a}, -C(O)OR^{a}, -C(O)NR^{a}R^{a}, -C(O)R^{b}, -OC(O)R^{b}, -NHC(O)R^{b} and -CN. More preferably, R^{PP} is a hydrogen, a lone pair of electrons resulting in a negative charge, o-chlorophenyl, p-chlorophenyl, a methyl group or a 2-cyanoethyl group. Further preferably, R^{PP} is a 2-cyanoethyl group.

When R^{P} is a phosphorous-based linkage to a nucleoside, nucleotide or polynucleotide or a derivative or analogue thereof, the nucleoside, nucleotide or polynucleotide or a derivative or analogue thereof may be any of the nucleosides, nucleotides or polynucleotides or derivatives or analogues thereof that may be represented by Ⓝ, such that Ⓝ may comprise multiple nucleosides, nucleotides or polynucleotides or derivatives or analogues thereof connected by phosphorous-based linkages.

R^{B} is an optionally substituted natural or non-natural nucleobase or a derivative or analogue thereof. Natural nucleobases include adenine, cytosine, guanine, thymine, and uracil. Non-natural nucleobases are well known in the art, and include, for example, analogues of the natural nucleobases such as 2-thiothymine, isomers of the natural nucleobases such as isoguanine, isocyanine and 5-aza-7-deazaguanine, and other groups used in the art as synthetic nucleobases, such as xanthine. For example, non-natural nucleobases include 2-thiothymine, isoguanine, isocytosine, 1-methylcytosine, 5-aza-7-deazaguanine, 6-amino-5-nitropyridin-2-one, 1-methylcytosine, xanthine, hypoxanthine, 7-methylguanine, 5,6-dihydrouracil, 5-methylcytosine, 5-hydroxymethylcytosine, 2,6-diaminopurine, 6,8-diaminopurine, fluorocytosine, fluorouracil, mercaptopurine, thioguanine, 5-hydroxymethyl uracil, 5-carboxycytosine, and fluorescent nucleobases such 2-aminopurine and tricyclic cytosines tC, tCO and tCnitro.

Derivatives or analogues of natural and non-natural nucleobases include protected natural and non-natural nucleobases - i.e. nucleobases that have been modified to include protecting groups in their chemical structure.

Preferably, R^{B} is a protected natural or non-natural nucleobase. More preferably, R^{B} is a protected natural nucleobase.

When R^{DR} is -OR^{a} and the R^{a} group and one of the R¹ groups are joined together to form a bridging motif, the moiety represented by formula (N-1) or formula (N-2) is preferably a moiety represented by formula (N-L1) or formula (N-L2): 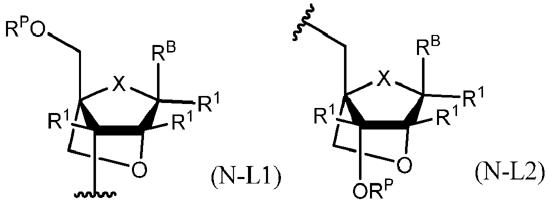 wherein X, each R¹, R^{P} and R^{B} are as defined above.

### Attachment to a support

As explained above, provided herein are methods which comprise the use of a support-attached nucleoside, nucleotide, polynucleotide or derivative thereof which is a compound of formula (I) attached to a solid support. In some embodiments the invention further provides support-attached compounds of formula (I) or related formulae such as formula (Ia) described herein. Such compounds are attached to a solid support at group Ⓐ of the compound.

In such embodiments wherein the compound is attached to a support, preferably Ⓐ is chemically attached to the support. Preferably, Ⓐ is covalently attached to a solid support. More preferably, Ⓐ is covalently attached to a linking group and the linking group is covalently attached to the solid support. This provides a compound (e.g. a compound of formula (I)) that is stably attached to the solid support.

In some embodiments Ⓐ is a moiety of formula (A') below.

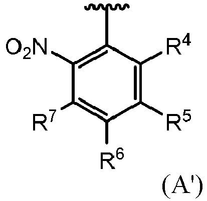

In formula (A') one of R⁴, R⁵, R⁶ and R⁷ is replaced by a moiety -L-Z wherein L is a linking group and Z is a solid support, and the remaining groups of R⁴, R⁵, R⁶ and R⁷ are as described below with respect to formulae (A-1) and (A-2).

More preferably, the linking group is attached to the 4-position or the 5-position of the 2-nitrobenzyl group represented by Ⓐ (when numbered such that the nitro group is at the 2-position relative to the -C(R³)-Q- moiety of formula (I), as indicated above) - that is, the linking group is preferably para or meta to the nitro group on the 2-nitrobenzyl group.

Further preferably, Ⓐ is a moiety of formula (A-1) or formula (A-2).

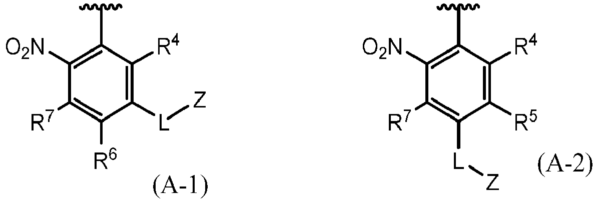

Preferably, Ⓐ is a moiety of formula (A-1).

In formula (A-1) and (A-2), R⁴, R⁵, R⁶ and R⁷ are each independently selected from hydrogen and a substituent which may be an electron-donating group. In formula (A-1) and (A-2), R⁴, R⁵, R⁶ and R⁷ are typically each independently selected from hydrogen, halogen, optionally substituted C₁ to C₄ alkyl, -OR^{a}, -SR^{a}, -NR^{a}R^{a}, -C(O)OR^{a}, - C(O)NR^{a}R^{a}, -C(O)R^{b}, -OC(O)R^{b} and -NHC(O)R^{b}, wherein R^{a} and R^{b} are as defined above.

Preferably, R⁴, R⁵, R⁶ and R⁷ are each independently selected from hydrogen, fluorine, optionally substituted C₁ to C₄ alkyl, -OR^{a}, -SR^{a}, -NR^{a}R^{a}, -OC(O)R^{b} and -NHC(O)R^{b}. More preferably, R⁴, R⁵, R⁶ and R⁷ are each independently selected from hydrogen, -OR^{a}, -SR^{a}, -NR^{a}R^{a}, -OC(O)R^{b} and -NHC(O)R^{b}. Still more preferably R⁴, R⁵, R⁶ and R⁷ are each independently selected from hydrogen, -OR^{a}, and -OC(O)R^{b}. Further preferably, R⁴, R⁵, R⁶ and R⁷ are each independently selected from hydrogen and methoxy. Without being bound by theory, the inventors believe that when one or more of R⁴, R⁵, R⁶ and R⁷ are electron donating groups, the linker is more easily cleaved using photoexcitation. When R⁴, R⁵, R⁶ and/or R⁷ is hydrogen, the synthesis of the compound of formula (I) may be more facile.

Preferably, at least one of R⁴, R⁵, R⁶ and/or R⁷ is hydrogen. For example, one, two or three of R⁴, R⁵, R⁶ and/or R⁷ is hydrogen. Preferably, one of R⁴ and R⁷ is hydrogen and the other of R⁴ and R⁷ is selected from hydrogen, -OR^{a}, and -OC(O)R^{b}. Preferably, R⁵ or R⁶ is hydrogen. For example, in some embodiments one of R⁴ and R⁷ is hydrogen and the other of R⁴ and R⁷ is selected from hydrogen, -OR^{a}, and -OC(O)R^{b} and R⁵ or R⁶ is hydrogen or methoxy.

When the compound is attached to a solid support (Z) by a linking group (L), as in formula (A'), (A-1) and (A-2) above, L is the linking group and Z is the solid support.

The linking group L may be any suitable linking group for linking a polynucleotide to a solid support. Preferably, the linking group is or comprises a C₁ to C₂₀ alkylene group, a C₂ to C₂₀ alkenylene group, a C₂ to C₂₀ alkynylene group and/or a nucleotide or a polynucleotide, wherein said alkylene, alkenylene or alkynylene group is optionally interrupted by and/or terminated in one or more groups selected from: a heteroatom or other heterogroup as described herein; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered saturated or partially unsaturated heterocyclic group; and wherein the linking group is optionally further substituted. Suitable heteroatoms and heterogroups include -O-, -S-, -SO-, -SO₂-, -NR^{z}-, -C(O)NR^{z}, etc, wherein R^{z} is H or methyl, preferably H. An exemplary alkylene group which is interrupted by and/or terminated in an -O- atom is an alkylene glycol group and therefore the linking group may comprise or consist of a polyalkylene glycol such as polyethylene glycol (PEG). Typically the linking group is not linked to a biotin or streptavidin moiety.

More preferably, the linking group is or comprises a C₁ to C₁₀ alkylene group, a C₂ to C₁₀ alkenylene group, and/or a polynucleotide, wherein said alkylene or alkenylene group is optionally interrupted by and/or terminated in one or more groups selected from: - O-, -S-, -SO-, -SO₂-, -NR^{z}-, -C(O)-, -C(O)NR^{z}, wherein R^{z} is H or methyl; a phosphite group; a phosphate group; a C₆ aryl group; a C₅ to C₆ carbocyclyl group; a 5- to 6-membered heteroaryl group; and a 5- to 6-membered saturated or partially unsaturated heterocyclic group. Still more preferably, the linking group is or comprises a C₁ to C₁₀ alkylene group and/or a polynucleotide, wherein said alkylene group is optionally interrupted by and/or terminated in one or more groups selected from: -O-, -NR^{z}-, -C(O)-, -C(O)NR^{z}-, wherein R^{z} is H or methyl; a C₆ aryl group; a C₅ to C₆ carbocyclyl group; a 5-to 6-membered heteroaryl group; and a 5- to 6-membered saturated or partially unsaturated heterocyclic group. Preferably when the linking group comprises a 5- to 6-membered heteroaryl group the heteroaryl group is a triazole or imidazole, preferably triazole.

The linking group may comprise a 1,2,3-triazole, polyethylene glycol, a phosphonate group, a phosphoramidite group and/or a succinyl group.

In some embodiments the linking group comprises a moiety of form: -A-B-C-wherein A is a C₁ to C₅ alkyl group which alkyl group is unsubstituted or substituted as described herein, preferably unsubstituted; B is a cyclic group selected from a C₆ aryl group; a C₅ to C₆ carbocyclyl group; a 5- to 6-membered heteroaryl group; and a 5- to 6-membered saturated or partially unsaturated heterocyclic group; and C is a C₁ to C₅ alkyl group which alkyl group is unsubstituted or substituted as described herein, preferably unsubstituted.

Preferably, when the linking group comprises a moiety of form: -A-B-C-, A is an unsubstituted C₁ to C₄ alkyl group; B is a cyclic group selected from a C₆ aryl group; a C₅ to C₆ carbocyclyl group; a 5- to 6-membered heteroaryl group; and a 5- to 6-membered saturated or partially unsaturated heterocyclic group; and C is an unsubstituted C₁ to C₄ alkyl group.

More preferably, when the linking group comprises a moiety of form: -A-B-C-, A is an unsubstituted C₁ to C₄ alkyl group; B is a cyclic group selected from triazole, benzene, cyclohexane, piperidine, pyridazine, pyridine, thiazole and imidazole, preferably triazole; and C is an unsubstituted C₁ to C₄ alkyl group.

In some embodiments the linking group comprises a moiety of the form: 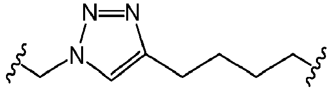 wherein the wavy lines indicate the points of attachment to the rest of the molecule.

Preferably, the linking group is attached to the solid support via a phosphoramidite ((RO)₂PNR₂) or phosphonate group, more preferably a phosphoramidite group. Examples of suitable phosphoramidite groups include -O-P(OR^{q})(NR^{p})₂ groups wherein R^{q} is a C₁ to C₄ alkyl which is substituted with a substituent such -CN and each R^{p} which may be the same or different is an optionally substituted C₁ to C₆ alkyl group, preferably an unsubstituted C₂ to C₄ alkyl group such as isopropyl. An example of a suitable phosphoramidite group is therefore 2-cyanoethyl diisopropylphosphoramidite (OCEP). Reaction of such a group with a reactive functional group on the solid support attaches the linking group and therefore the linked compound to the support.

### Specifically disclosed embodiments

Preferably in formula (I):
- Q is an oxygen atom;
- each R² is independently hydrogen, methyl, C₁ fluoroalkyl or fluorine;
- R³ is methyl, ethyl or C₁ to C₂ fluoroalkyl;
- Ⓐ is a 2-nitrobenzyl group, wherein said 2-nitrobenzyl group is optionally substituted with 1, 2 or 3, preferably 1 or 2 groups selected from C₁ to C₄ alkyl, -OR^{a}, -SR^{a}, -NR^{a}R^{a}, -OC(O)R^{b} and -NHC(O)R^{b};
- each R^{a} is independently selected from hydrogen, optionally substituted C₁ to C₂ alkyl and optionally substituted C₁ to C₂ alkoxyl;
- each R^{b} is independently selected from hydrogen and an optionally substituted C₁ to C₄ alkyl group;
- Ⓝ is a moiety of formula (N-1) or formula (N-2) 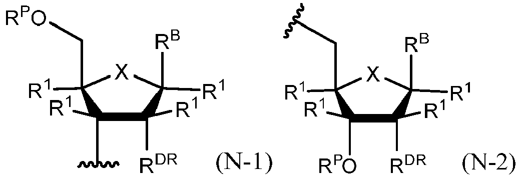 wherein:
   ∘ the wavy line indicates the point of attachment to the compound of formula (I);
   ∘ X is an oxygen atom, a nitrogen atom, a sulphur atom or -C(R^{a}R^{a})-;
   ∘ Each R¹ is independently selected from hydrogen, halogen, C₁ to C₂ alkyl, C₁ to C₂ haloalkyl and C₁ to C₂ alkoxyl.
   ∘ R^{DR} is a hydrogen, halogen, C₁ to C₂ alkyl or -OR^{a}.
   ∘ R^{P} is a hydrogen, hydroxyl protecting group, or a phosphodiester linkage to a nucleoside, nucleotide or polynucleotide or a derivative or analogue thereof.
   ∘ R^{B} is an optionally substituted natural or non-natural nucleobase
- and, when the compound of formula (I) is attached to a support as described herein, Ⓐ is a moiety of formula (A') 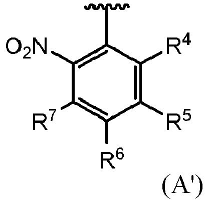 wherein one of R⁴, R⁵, R⁶ and R⁷ is replaced by a moiety -L-Z wherein L is a linking group and Z is a solid support, and the remaining groups of R⁴, R⁵, R⁶ and R⁷ are independently selected from hydrogen, C₁ to C₄ alkyl, -OR^{a}, -SR^{a}, -NR^{a}R^{a}, -OC(O)R^{b} and -NHC(O)R^{b}, wherein R^{a} and R^{b} are as defined above;

L is or comprises a C₁ to C₂₀ alkylene group, a C₂ to C₂₀ alkenylene group, a C₂ to C₂₀ alkynylene group and/or a nucleotide or a polynucleotide, wherein said alkylene, alkenylene or alkynylene group is optionally interrupted by and/or terminated in one or more groups selected from: a heteroatom or other heterogroup as described herein; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered saturated or partially unsaturated heterocyclic group; and wherein the linking group is optionally further substituted; and wherein the linking group is attached to the solid support via a phosphoramidite ((RO)₂PNR₂) or phosphonate group.

More preferably in formula (I):
- Q is an oxygen atom;
- each R² is independently hydrogen or methyl, preferably hydrogen;
- R³ is methyl, ethyl or CF₃; preferably methyl;
- Ⓐ is a 2-nitrobenzyl group, wherein said 2-nitrobenzyl group is optionally substituted with 1 or 2 groups selected from -OR^{a}, and -OC(O)R^{b}; more preferably Ⓐ unsubstituted apart from by the nitro group at the 2 position and optionally by an L-Z moiety as defined herein;
- each R^{a} is independently selected from hydrogen and methyl;
- each R^{b} is independently selected from hydrogen and methyl;.
- Ⓝ is a moiety of formula (N-1) or formula (N-2) 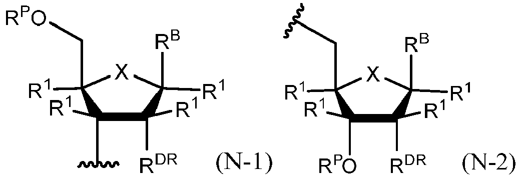 wherein:
   ∘ the wavy line indicates the point of attachment to the compound of formula (I);
   ∘ X is an oxygen atom;
   ∘ each R¹ is independently selected from hydrogen, fluorine, methyl and methoxy; preferably hydrogen
   ∘ R^{DR} is a hydrogen, hydroxy or methoxy; preferably hydrogen;
   ∘ R^{P} is a hydrogen, hydroxyl protecting group, or a phosphodiester linkage to a nucleoside, nucleotide or polynucleotide.
   ∘ R^{B} is an optionally substituted natural or non-natural nucleobase; preferably a natural nucleobase;
   and, when the compound of formula (I) is attached to a support as described herein,
   Ⓐ is a moiety of formula (A-1) or formula (A-2). 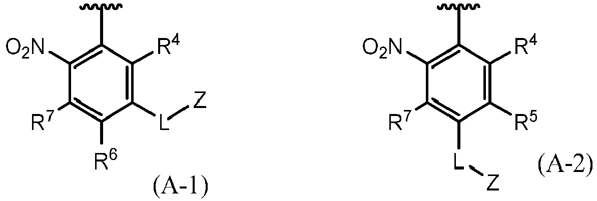
wherein R⁴, R⁵, R⁶ and R⁷ are independently selected from hydrogen, -OR^{a}, and -OC(O)R^{b} wherein R^{a} and R^{b} are as defined above;
L is or comprises a C₁ to C₁₀ alkylene group, a C₂ to C₁₀ alkenylene group, and/or a polynucleotide, wherein said alkylene or alkenylene group is optionally interrupted by and/or terminated in one or more groups selected from: -O-, -S-, - SO-, -SO₂-, -NR^{z}-, -C(O)-, -C(O)NR^{z}, wherein R^{z} is H or methyl; a phosphite group; a phosphate group; a C₆ aryl group; a C₅ to C₆ carbocyclyl group; a 5- to 6-membered heteroaryl group; and a 5- to 6-membered saturated or partially unsaturated heterocyclic group; and
wherein the linking group is attached to the solid support via a phosphoramidite group.

*Method of producing a polynucleotide or analogue or derivative thereof.*

As discussed above, the invention provides a method of producing a polynucleotide comprising:
(i) providing a support-attached nucleoside, nucleotide, polynucleotide or derivative or analogue thereof, which is a compound of formula (I) attached to a solid support at Ⓐ, 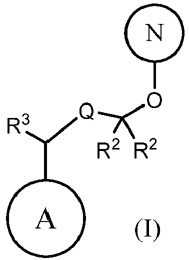 wherein Ⓝ, Q, R², R³ and Ⓐ are as defined above;
(ii) increasing the chain length of the support-attached nucleoside, nucleotide, polynucleotide or derivative or analogue thereof by introducing one or more additional nucleoside units, nucleotide units and/or analogues or derivatives thereof; and
(iii) cleaving the resulting polynucleotide from the solid support by photo-illuminating the compound of formula (I).

Step (i) of the method of the present invention may comprise providing a previously prepared support-attached nucleoside, nucleotide, polynucleotide or derivative or analogue thereof, which is a compound of formula (I) attached to a solid support as described herein.

Alternatively, step (i) may comprise preparing a support-attached nucleoside, nucleotide, polynucleotide or derivative or analogue thereof, which is a compound of formula (I) attached to a solid support. Thus, step (i) may comprise attaching a compound of formula (I) to a solid support.

Alternatively, step (i) may comprise preparing the compound of formula (I) attached to a solid support at Ⓐ from a compound of formula (Ia) attached to a solid support at Ⓐ; wherein formula (I) is 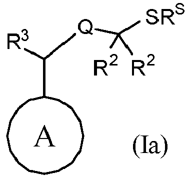 and wherein Q, R², R³ and Ⓐ are as defined above, and R^{S} is a C₁ to C₄ alkyl.

Step (i) may thus comprise attaching a compound of formula (Ia) to a solid support at Ⓐ. For example, step (i) of the method of the present invention may comprise:
(a) providing a compound of formula (Ia), 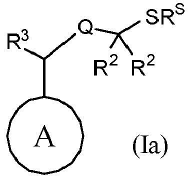 wherein Q, R², R³ and Ⓐ are as defined above, and R^{S} is a C₁ to C₄ alkyl; and
(b) reacting the compound of formula (Ia) with a nucleoside, nucleotide, polynucleotide or derivative or analogue thereof; wherein preferably the nucleoside, nucleotide, polynucleotide or derivative or analogue thereof is represented by formula (N-1) or (N-2) as defined above;
thereby forming a compound of formula (I).

Preferably, R^{S} is methyl.

Step (ii) of the method of the present invention preferably comprises performing the following step one or more times: contacting the support-attached nucleoside, nucleotide, polynucleotide, or derivative or analogue thereof with a nucleoside or a derivative thereof, thereby forming a phosphorous-based linkage.

The nucleoside or a derivative thereof may be a nucleoside phosphoramidite, a nucleoside H-phosphonate or a nucleoside phosphotriester, preferably an activated nucleoside phosphoramidite, an activated nucleoside H-phosphonate or an activated nucleoside phosphotriester. A nucleoside phosphoramidite may be activated by reaction with tetrazole or a derivative thereof as an activator; for example, a group of the phosphoramidite (such as a diisopropylamino group of the phosphoramidate) may be protonated by acidic tetrazole or a derivative thereof. A nucleoside H-phosphonate may be activated by reaction with a chlorinating agent such as pivaloyl chloride. A nucleoside nucleoside phosphotriester may be activated by reaction with a coupling agent such as 1-(mesitylsulfonyl)-3-nitro-1H-1,2,4-triazole.

Preferably, in step (ii) the nucleoside or a derivative thereof is an activated nucleoside phosphoramidite. Preferably, the activated nucleoside phosphoramidite has an activated phosphoramidite group at the 3' position or the 5' position. More preferably, the activated nucleoside phosphoramidite has an activated phosphoramidite group at the 3' position. Preferably, the activated nucleoside phosphoramidite with an activated phosphoramidite group at the 3' position has a hydroxyl protecting group at the 5' position. Preferably, the activated nucleoside phosphoramidite with an activated phosphoramidite group at the 5' position has a hydroxyl protecting group at the 3' position. Preferably, the activated phosphoramidite group is represented by formula (PH-A): 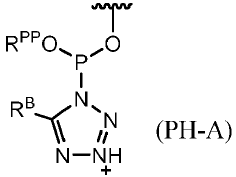 R^{PP} and R^{b} are as defined herein.

The phosphorous-based linkage may be a phosphodiester linkage, a phosphotriester linkage such as an alkyl phosphotriester linkage, a phosphite-triester linkage such as an alkyl phosphite-triester linkage, a phosphorothioate linkage, a phosphorodithioate linkage, or an alkylphosphonate linkage such as a methyl phosphonate linkage. Preferably, the phosphorous-basedlinkage is a phosphite-triester linkage. More preferably, the phosphorous-based linkage is a 2-cyanoethyl phosphite-triester linkage.

Preferably, step (ii) comprises performing the following steps one or more times: providing a nucleoside phosphoramidite; activating the nucleoside phosphoramidite; and contacting the support-attached nucleoside, nucleotide, polynucleotide, or derivative or analogue thereof with the activated nucleoside phosphoramidite, thereby forming a phosphite triester linkage.

The nucleoside phosphoramidite preferably has a phosphoramidite group at the 3' position or the 5' position. More preferably, the nucleoside phosphoramidite has a phosphoramidite group at the 3' position. Preferably, the nucleoside phosphoramidite with a phosphoramidite group at the 3' position has a hydroxyl protecting group at the 5' position. Preferably, the nucleoside phosphoramidite with a phosphoramidite group at the 5' position has a hydroxyl protecting group at the 3' position. Preferably, the phosphoramidite group is represented by formula (PH): 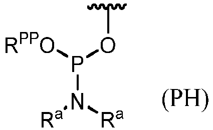 R^{PP} and R^{a} are as defined herein.

Step (ii) may optionally further comprise oxidising the phosphite triester linkage to form a phosphotriester linkage.

Step (ii) may optionally further comprise removing a hydroxyl protecting group.

More preferably, step (ii) comprises performing the following steps (a) to (e) one or more times:
(a) providing a nucleoside having: (i) a phosphoramidite group at the 3' position and a hydroxyl protecting group at the 5' position; or (ii) a phosporamidite group at the 5' position and a hydroxyl protecting group at the 3' position;
(b) activating the nucleoside phosphoramidite of step (a);
(c) contacting the support-attached nucleoside, nucleotide, polynucleotide, or derivative or analogue thereof with the activated nucleoside phosphoramidite, thereby forming a phosphite triester linkage;
(d) oxidising the phosphite triester linkage to form a phosphotriester linkage; and
(e) removing the hydroxyl protecting group from the terminal nucleoside of the resulting support-attached polynucleotide.

In step (a), the nucleoside preferably has a phosphoramidite group at the 3' position and a hydroxyl protecting group at the 5' position.

Step (b) typically comprises contacting the nucleoside phosphoramidite with a suitable activating agent. Any suitable activating agent can be used. Typically the activating agent is tetrazole or a derivative thereof. Typically the activating agent and nucleoside phosphoramidite are contacted in an organic solvent such as acetonitrile.

Step (c) may be conducted by flowing the activated nucleoside phosphoramidite of step (b) through a column containing the support-attached nucleoside, nucleotide, polynucleotide, or derivative or analogue thereof.

Step (d) typically comprises oxidising the phosphite triester using a suitable oxidising agent. Any suitable oxidising agent can be used. Typically the oxidising agent is iodine. Iodine may be used in reaction with suitable solvents e.g. with water and pyridine (e.g. at a concentration of about 0.01 to 0.02 M iodine in water/pyridine/THF 2/20/78).

Step (e) may be conducted by contacting the resulting support-attached polynucleotide of step (d) with a suitable reagent. Typically, deprotection comprises contacting the support-attached polynucleotide with concentrated aqueous base such as aqueous ammonia. The aqueous solution may be removed by evaporation.

Step (ii) may comprise repeating said reaction step(s) at least once, at least twice, at least 5 times, at least 10 times, at least 20 times, at least 30 times, at least 40 times, at least 50 times, at least 100 times, at least 150 times, at least 200 times, at least 500 times, at least 1000 times, or more. Accordingly, step (ii) may comprise forming a polynucleotide of length at least 2, at least 5, at least 10, at least 20, at least 30, at least 40, at least 50, at least 100, at least 150, at least 200, at least 500, at least 1000 monomer units, or more. Those skilled in the art will appreciate that at each repeat the nucleoside or derivative thereof attached to the support-attached nucleoside provided in step (i) may be the same or different. Accordingly, the sequence of the polynucleotide synthesized in the method may be determined by the user of the method, e.g. the operator of equipment configured for the synthesis of polynucleotides.

Preferably, the method of the present invention comprises one or more steps of deprotecting the support-attached polynucleotide. Preferably, these steps are performed after step (ii) and before step (iii). Preferably, deprotecting the support-attached polynucleotide comprises removing protecting groups from one or more nucleobases and/or one or more phosphorous-based linkages present in the support-attached polynucleotide. Deprotection may be achieved by treatment of the support-attached polynucleotide with a suitable reagent depending on the protecting group used.

For example, benzoyl, isobutryl and dimethylformamidyl protecting groups typically used to protect nucleobases can be removed using concentrated ammonium hydrocide. Ultramild protecting groups typically used to protect nucleobases such as phenoxyacetyl, acetyl and isopropyl phenyoxyacetyl groups can be removed using a methanolic solution of potassium carbonate, or a mixture of aqueous ammonia and aqueous methylamine. Cyanoethyl phosphotriester groups can be used to protect the phosphodiester backbone and can be removed using concentrated ammonium hydroxide. Phosphate groups can be protected as methyl triesters and removed using thiophenol.

In step (iii) the resulting polynucleotide is cleaved from the solid support by photo-illuminating the compound of formula (I).

Step (iii) of the present invention preferably comprises photo-illuminating the compound of formula (I) using UV light at a wavelength of about 300 to about 500 nm, preferably about 325 to about 475 nm, more preferably about 350 to about 450 nm. Photoillumination may be carried out for any suitable time scale depending on the power of the light source used. For example, a power of from about 100 W to about 5000 W, e.g. from about 200 W to about 1000 W such as about 500 W can be applied for from about 1 minute to about 10 hours, such as from about 10 minutes to about 1 hour e.g. for about 30 minutes. Suitable illumination can be provided by an Hg lamp. Selecting appropriate illumination conditions is routine for those skilled in the art. Appropriate illumination conditions can be determined by monitoring the extent of cleavage and selecting conditions which result in substantial or complete cleavage in the minimum time possible without causing damage to the synthesized polynucleotide, according to the equipment used. The product of the polynucleotide synthesis can be assessed using techniques such as e.g. NMR and mass spectroscopy to monitor the reaction.

The method of the invention can be conducted at any appropriate temperature and under routine solvent conditions that are readily accessible to those skilled in the art. Typically, reactions are conducted at from about 10 °C to about 100 °C, such as from about 20 °C to about 50 °C such as from about 25 °C to about 30 °C. Often reactions can be conducted at room temperature.

The compound of formula (I) used in the method of the present invention is compatible with a wide range of well-known methods of polynucleotide synthesis, including phosphoramidite methods, H-phosphonate methods and phosphotriester methods. Thus, the method of the present invention includes phosphoramidite methods of polynucleotide synthesis, H-phosphonate methods of polynucleotide synthesis and phosphotriester methods of polynucleotide synthesis. Preferably, the method of the present invention is a phosphoramidite method of polynucleotide synthesis.

### Further Compounds

Also provided herein is a support-attached nucleoside, nucleotide, or derivative or analogue thereof, which is a compound of formula (I) attached to a solid support at Ⓐ, 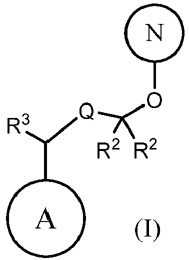 wherein Q, R², R³ and Ⓐ are as defined above and Ⓐ is a nucleoside, nucleotide or derivative or analogue thereof. Preferably, Ⓐ is represented by formula (N-1) or formula (N-2) as defined above, wherein R^{P} is a hydrogen, hydroxyl protecting group, phosphoryl group or a salt or acid therof, diphosphoryl group or a salt or acid thereof or triphosphoryl group or a salt or acid thereof.

This compound comprises a single monomer unit of a polynucleotide and is useful as a starting point for polynucleotide synthesis. In particular, this compound may be provided in step (i) of the method of the present invention.

The present invention also provides a compound of formula (I), 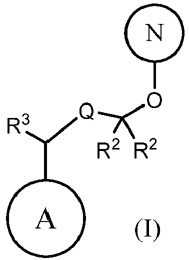 wherein Q, R², R³ and Ⓐ are as defined above, Ⓐ is represented by formula (N-1) or formula (N-2) as defined above, and Ⓐ is optionally attached to a solid support. Ⓐ is preferably represented by formula (N-1) as defined above.

This compound comprises a nucleoside, nucleotide or polynucleotide or a derivative or analogue thereof which is attached to the photocleavable linker of formula (I) at the 3' position or the 5' position, preferably at the 3' position. Thus, this compound is ideal for use in polynucleotide synthesis in the 3' to 5' direction or the 5' to 3' direction.

When Ⓐ is attached to a solid support, this compound may be provided in step (i) of the method of the present invention.

When Ⓐ is not attached to a solid support, this compound may be used to produce a compound of formula (I) which is attached to a solid support at Ⓐ. Alternatively, this compound is also useful as a blocked nucleotide compound. In particular, the photocleavable group of this compound could serve as a protecting group that blocks the reactivity of the nucleoside hydroxyl group to which it is attached, but which can be removed at will using photoexcitation. Removal of the photocleavable group produces a nucleoside, nucleotide or polynucleotide or a derivative or analogue thereof having a free hydroxyl group, which is able to undergo a variety of reactions (such as a coupling reaction with a polynucleotide chain to increase the chain length).

Thus, this compound could be present in solution with a mixture of other compounds but be inactive and inert until, at a desired point in time, the compound is activated using photoexcitation.

The present invention also provides a compound of formula (Ia) which is attached to a solid support at Ⓐ, 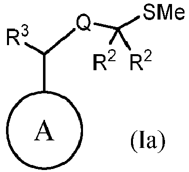 wherein Q, R², R³ and Ⓐ are as defined above.

This compound is useful in producing a compound of formula (I) which is attached to a solid support at Ⓐ.

### Solid support

In embodiments provided herein, a compound provided herein may be attached to a solid support. For example, in some embodiments the compound of formula (I) or formula (Ia) is attached to a solid support at the group Ⓐ of said compound.

Any suitable solid support may be used.

Preferably, the solid support comprises a glass, silica, ceramic, or a polymeric resin. More preferably, the solid support comprises controlled pore glass (CPG) or polystyrene. CPG may be treated with an appropriate surface treatment to introduce one or more functional groups. Suitable surface treatments include silanes such as (3-aminopropyl)triethoxysilane to give aminopropyl CPG. Such functionalised CPG can be derivatised by reacting the amino moiety of the aminopropyl group with a suitable moiety such as an ester group. Suitable polystyrene materials are typically low-swellable and/or cross-linked. For example, polystyrene can be obtained by polymerization of divinylbenzene, styrene, and 4-chloromethylstyrene in the presence of a porogeneous agent. The macroporous chloromethyl MPPS obtained may be converted to aminomethyl MPPS.

Typically the solid support is not functionalised with a protein such as streptavidin.

Preferably, the solid support comprises particles having a diameter of from about 1 µm to about 10000 µm, more preferably from about 5 µm to about 1000 µm, further preferably from about 10 µm to about 500 µm such as from about 50 to about 250 µm.

Preferably, the solid support material particles are porous. Preferably, when the solid support material is porous the pore size is from about 1 to about 1000 nm e.g. from about 10 to about 100 nm such as about 50 nm.

Preferably, the support material is or comprises beads. Such beads may be magnetic e.g. may comprise a magnetic material. Beads may be ferrimagentic or paramagnetic. Beads may comprise an iron oxide core.

The use of beads may be advantageous when the synthesized polynucleotide is to be used in sequencing technologies, e.g. in droplet-based sequencing as described herein.

Preferably, the support material is contained in a column. The column may have a bed volume of from about 100 µL to about 100 L; e.g. from about 1 mL to about 1 L. Any suitable bed volume can be used depending on the scale of the synthesis envisaged.

Accordingly, provided herein is a solid support column for solid-phase polynucleotide synthesis, comprising a support-attached nucleoside, nucleotide, polynucleotide or a derivative or analogue thereof which is a compound of formula (I) attached to a solid support at Ⓐ, wherein Ⓝ, Q, R², R³ and Ⓐ are as defined in any one of the preceding claims.

### Products of the disclosed methods

The disclosed methods may be used to produce a polynucleotide or an analog or derivative thereof. Accordingly, also provided herein is a polynucleotide or derivative or analog thereof obtainable or obtained by a method provided herein.

The term "polynucleotide" refers to a single or double stranded covalently-linked sequence of nucleotides in which the 3' and 5' ends on each nucleotide are joined by phosphorous-based linkages. The phosphorous-based linkages present in products of the disclosed methods may be phosphodiester bonds, as in natural polynucleotides, or may be non-natural phosphorous-based bonds such as: phosphotriester linkages, for example, alkyl phosphotriester linkages such as methyl phosphotriester linkages and ethyl phosphotriester linkages; phosphorothioate linkages; phosphorodithioate linkages; and alkylphosphonate linkages such as methyl phosphonate linkages.

The polynucleotide may be made up of deoxyribonucleotide bases or ribonucleotide bases. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as "polynucleotides" are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides" and may comprise primers for use in manipulation of DNA such as via polymerase chain reaction (PCR).

The synthesized polynucleotide may comprise any combination of any nucleotides. The nucleotides can be naturally occurring or artificial. One or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light and are the primary cause of skin melanomas. One or more nucleotides in the polynucleotide may be modified, for instance with a label or a tag, for which suitable examples are known by a skilled person. The polynucleotide may comprise one or more spacers. A nucleotide typically contains a nucleobase, a sugar and at least one phosphate group as described in more detail herein. The nucleobase and sugar form a nucleoside. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The sugar is preferably a deoxyribose. The polynucleotide preferably comprises the following nucleosides: deoxyadenosine (dA), deoxyuridine (dU) and/or thymidine (dT), deoxyguanosine (dG) and deoxycytidine (dC). The nucleotide is typically a ribonucleotide or deoxyribonucleotide. The nucleotide typically contains a monophosphate, diphosphate or triphosphate. The nucleotide may comprise more than three phosphates, such as 4 or 5 phosphates. Phosphates may be attached on the 5' or 3' side of a nucleotide. The nucleotides in the polynucleotide may be attached to each other in any manner. The nucleotides are typically attached by their sugar and phosphate groups as in nucleic acids. The nucleotides may be connected via their nucleobases as in pyrimidine dimers.

Nucleotides comprised in the polynucleotide may include, but are not limited to, adenosine monophosphate (AMP), guanosine monophosphate (GMP), thymidine monophosphate (TMP), uridine monophosphate (UMP), 5-methylcytidine monophosphate, 5-hydroxymethylcytidine monophosphate, cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), deoxycytidine monophosphate (dCMP) and deoxymethylcytidine monophosphate. Nucleotides are preferably selected from AMP, TMP, GMP, CMP, UMP, dAMP, dTMP, dGMP, dCMP and dUMP. A nucleotide may be abasic (i.e. lack a nucleobase). A nucleotide may also lack a nucleobase and a sugar (i.e. may be a C3 spacer).

Typically the polynucleotide consists or comprises DNA and/or RNA, preferably DNA.

The polynucleotide can be at least 10, at least 50, at least 100, at least 150, at least 200, at least 250, at least 300, at least 400 or at least 500 nucleotides or nucleotide pairs in length.

### Applications of polynucleotides produced by the disclosed methods

The polynucleotides provided by the disclosed methods and provided herein have a multitude of potential applications. The applications are not particularly limited and the polynucleotides provided here and by the methods disclosed herein can be used in any application for which polynucleotides are required.

In some embodiments the methods provided herein are capable of providing polynucleotides with a very high degree of purity. Accordingly, the polynucleotides provided herein are particularly useful in applications wherein high purity is necessary. Preferably, the purity of the polynucleotides obtained in the disclosed methods is at least 99%, such as at least 99.9%, e.g. at least 99.99% more preferably at least 99.999% or more.

One application of the polynucleotides provided herein is in PCR (polymerase chain reaction). Methods for conducting PCR are well known to those skilled in the art. For example, in one embodiment conducting PCT comprises contacting primers complementary to a target polynucleotide with the target polynucleotide under conditions such that a polymerase can catalyse chain extension thereby amplifying the target polynucleotide. The polynucleotides provided herein are particularly useful as primers for PCR due to their high degree of purity.

Another application of the polynucleotides provided herein is in the preparation of therapeutic polynucleotides. Therapeutic polynucleotides are being investigated for use in treating various different pathological conditions and have particular applications as anticancer medications and as antivirals.

Further applications of the polynucleotides provided herein include in next generation sequencing technologies. For example, the polynucleotides provided herein can be used as barcodes in single cell sequencing techniques such as drop-seq sequencing, discussed below.

### Single cell sequencing techniques

The method of the present invention can be advantageously used to produce support-attached polynucleotides for use in methods of sequencing the genomes of cells, and in particular in a cell sequencing technique known as the 'drop-seq' technique. Similarly, some of the compounds of the present invention can advantageously be used in methods involving the drop-seq technique.

In standard drop-seq techniques, a microfluidic device is used to create an emulsion of water droplets in oil in which each water droplet contains at most a single biological cell (e.g. a mammalian e.g. human cell or a bacterial cell) and a barcoded solid support, such as a barcoded bead, which is attached to a polynucleotide sequence defining a polynucleotide "barcode". The support attached polynucleotides of the invention can be used in this manner.

In more detail, support-attached polynucleotides for use in drop-seq sequencing typically comprise a molecular index for identifying cellular target polynucleotides; a polynucleotide "barcode" and a universal sequence. The support-attached polynucleotides typically comprises a 3'-poly T end - i.e. at the 3' ends of the polynucleotides, there are several e.g. from about 5 to about 50 such as from about 10 to about 40 e.g. about 30 nucleotide units in a row having thymine as their nucleobase. The molecular index may comprise from about 5 to about 15 nucleotides. The barcode may comprise from about 4 to about 50 such as from about 5 to about 30 e.g. about 6 to about 20 e.g. about 8 to about 15 monomer units. The universal sequence can be of any desired length.

The cells typically contain mRNA having a 5'-poly A region - i.e. at the 5' end of the mRNA there are several nucleotides in a row having adenine as their nucleobase. Typically, in the drop-seq technique, the cells are lysed and their mRNA is released into their respective droplets. The 5'-poly A regions of the mRNA strands hybridise to the 3'-poly T ends of the support-attached polynucleotides, thereby capturing the mRNA strands from a single cell onto a single solid support. The resulting support-attached polynucleotide-mRNA assemblies are often referred to as 'STAMPs' - Single cell Transcriptomes Attached to MicroParticles.

The emulsion may then be then disrupted, releasing the STAMPS, which may be used to create a transcriptome library. The STAMPS can be identified by the barcode on the solid support, and can undergo reverse transcription, PCR and sequencing to determine the mRNA strand sequences.

Previously, the support-attached polynucleotides with 3'-poly T ends used in drop-seq techniques have been synthesised directly on the solid support using solid-phase polynucleotide synthesis in the 5' to 3' direction, in order to yield free 3' ends available for hybridising with mRNA strands.

However, the compounds and methods of the present invention may be used to create support-attached polynucleotides for use in drop-seq techniques that are attached to the barcoded solid support at the 3' end. This has several advantages, which include: (i) the reagents needed to produce the barcoded support-attached polynucleotides are much cheaper - in particular, the so-called 'standard' nucleotide phosphoramidites typically used in 3' to 5' direction solid-phase polynucleotide synthesis are around an order of magnitude cheaper than the so-called 'reverse nucleotide phosphoramidites' typically used in 5' to 3' direction synthesis; (ii) the coupling efficiency of 3' to 5' direction solid-phase polynucleotide synthesis is typically higher than in 5' to 3' direction synthesis; and (iii) the variety in commercially available 'standard' nucleotide phosphoramidites is much greater than that of commercially available 'reverse nucleotide phosphoramidites', which allows superior customisation and thus improvement of the polynucleotide for capturing mRNA strands.

These barcoded support-attached polynucleotides synthesised in the 3' to 5' direction can be used in drop-seq techniques in which the polynucleotides are cleaved from their barcoded supports prior to disruption of the emulsion, using photoexcitation to cleave the photocleavable linker. The subsequent reverse transcription and PCR steps can then be performed 'in droplet' to sequence the mRNA chains present in each of the individual water droplets.

Accordingly, provided herein is a method of sequencing a polynucleotide expressed by a cell, the method comprising providing a support attached polynucleotide as described herein wherein said polynucleotide is attached to said support by a photocleavable linker as described herein; contacting said support attached polynucleotide or the photocleaved product of said support attached polynucleotide with a target polynucleotide under conditions such that the support attached polynucleotide or photocleaved product hybridises to the target polynucleotide; enzymatically synthesizing a complement polynucleotide strand complementary to the target polynucleotide; and sequencing the synthesized complement polynucleotide strand or a polynucleotide strand complementary thereto, thereby determining the sequence of the target polynucleotide.

### Further embodiments of the invention

Also provided herein is a kit for preparing a polynucleotide, comprising:
- a solid support modified with a photocleavable linker e.g. with a compound of formula (Ia) as described herein; and
- reagents for synthesizing the polynucleotide, preferably wherein said reagents comprise a plurality of nucleotide monomers.

Preferably the solid support and photocleavable linker are as defined herein.

Further provided is an apparatus for performing polynucleotide synthesis, the apparatus comprising:
- a solid support modified with a photocleavable linker as defined herein; and
- a light source for illuminating the solid support thereby cleaving the photocleavable linker.

Preferably the solid support, light source and photocleavable linker are as defined herein.

### Methods

The compounds of the invention can be prepared by any suitable method. Detailed general synthetic routes for representative compounds of the invention are set out below and in the Examples.

In summary, compounds of the invention can typically be prepared in a reaction according to the following scheme:

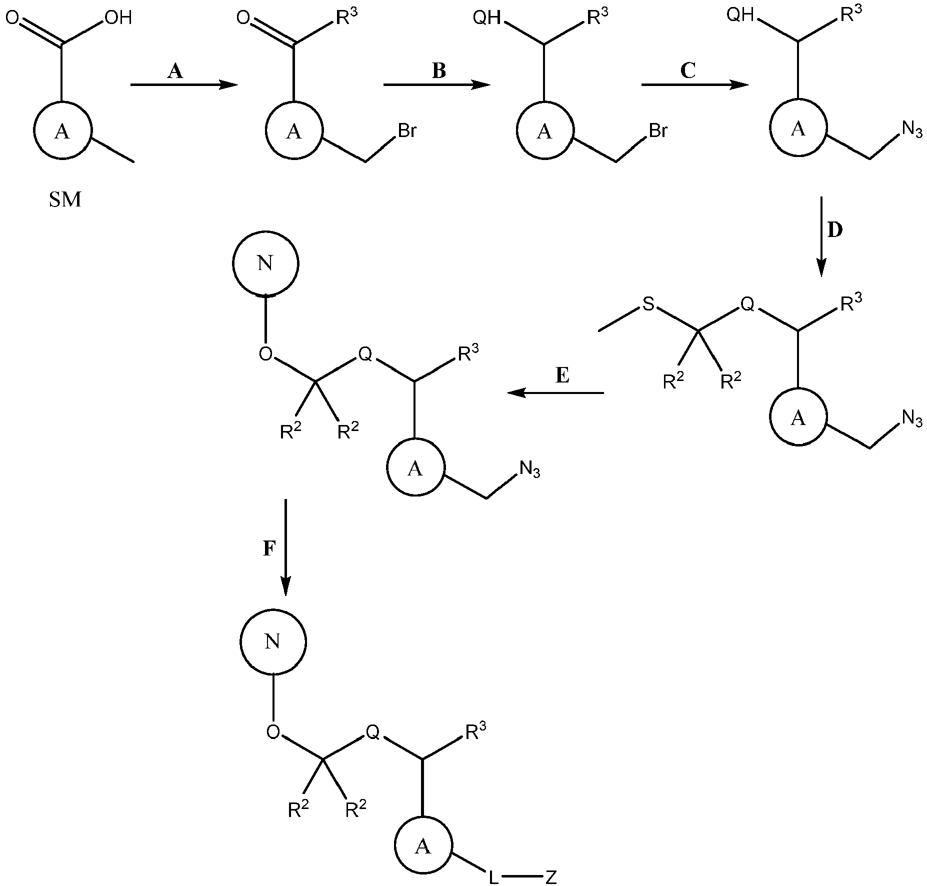

Starting material SM is readily available. **A:** Reaction of SM with e.g. SOCl₂ activates the carboxylic acid for reaction with e.g. a Grignard derivative of R³ before e.g. NBS is used to functionalise the alkyl on the (A) group. Other functionalisation chemistry is readily available. **B:** Reduction of the carboxylic acid can achieved using e.g. NaBH₄ to yield the alcohol (when Q is O). Standard conversion can be used to convert the alcohol to other functional groups when Q is other than O. **C:** NaN₃ can be used to convert the bromine to an azide prior to **(D:)** reaction with a sulfur-containing CR²₂-derivative to install a reactive group for reaction with a nucleotide, nucleoside or polynucleotide in step **E.** The azide can then be converted to a linking group for attachment to a solid support using routine chemistry e.g. via one or more nucleophilic substitution steps in **F.**

Detailed synthetic routes to exemplary compounds of the invention are set out below.

### Further Embodiments

The following are numbered aspects of the invention.
1. A method of producing a polynucleotide or an analogue or derivative thereof, comprising:
   (i) providing a support-attached nucleoside, nucleotide, polynucleotide or derivative or analogue thereof, which is a compound of formula (I) attached to a solid support at Ⓐ, 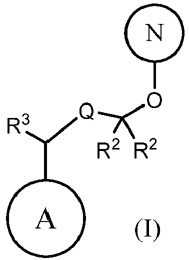 wherein:
      - Ⓝ is a nucleoside, nucleotide, polynucleotide or derivative or analogue thereof,
      - Q is an oxygen atom or a sulphur atom,
      - each R² is independently selected from hydrogen, methyl, ethyl, C₁ to C₂ haloalkyl and a halogen group,
      - R³ is methyl, ethyl or C₁ to C₂ haloalkyl, and
      - Ⓐ is a 2-nitrobenzyl group, wherein said 2-nitrobenzyl group is optionally substituted with 1, 2 or 3 groups independently selected from halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, wherein each R^{a} is independently selected from hydrogen, optionally substituted C₁ to C₂ alkyl and optionally substituted C₁ to C₂ alkoxyl and each R^{b} is independently selected from hydrogen and an optionally substituted C₁ to C₄ alkyl group;
   (ii) increasing the chain length of the support-attached nucleoside, nucleotide, polynucleotide or derivative or analogue thereof by introducing one or more additional nucleoside units, nucleotide units and/or analogues or derivatives thereof; and
   (iii) cleaving the resulting polynucleotide from the solid support by photo-illuminating the compound of formula (I).
2. The method of aspect 1, wherein
   - Q is an oxygen atom, and/or
   - each R² is independently selected from hydrogen, methyl, C₁ fluoroalkyl and fluorine; preferably each R² is hydrogen; and/or
   - R³ is methyl, ethyl or C₁ to C₂ fluoroalkyl; preferably R³ is methyl.
3. The method of aspect 1 or aspect 2, wherein Ⓐ is covalently attached to a linking group and the linking group is covalently attached to the solid support.
4. The method of aspect 3, wherein Ⓐ is represented by formula (A-1) or formula (A-2), 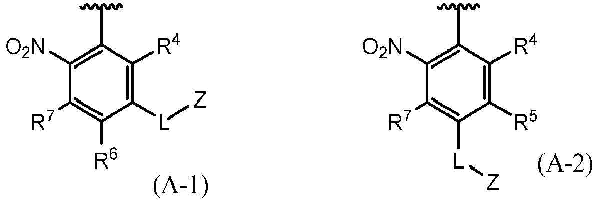 wherein:
   - R⁴, R⁵, R⁶ and R⁷ are each independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, wherein R^{a} and R^{b} are as defined in aspect 1;
   - L is the linking group; and
   - Z is the solid support.
5. The method of aspect 4, wherein R⁴, R⁵, R⁶ and R⁷ are each independently selected from hydrogen, fluorine, optionally substituted C₁ to C₄ alkyl, -OR^{a}, -SR^{a}, -NR^{a}R^{a}, -OC(O)R^{b} and -NHC(O)R^{b}, wherein R^{a} and R^{b} are as defined in aspect 1; wherein preferably R⁴, R⁵, R⁶ and R⁷ are each hydrogen or methoxy.
6. The method of any one of aspects 3 to 5, wherein the linking group comprises a C₁ to C₂₀ alkylene group, a C₂ to C₂₀ alkenylene group, a C₂ to C₂₀ alkynylene group and/or a nucleotide or a polynucleotide, wherein said alkylene, alkenylene or alkynylene group is optionally interrupted by and/or terminated in one or more groups selected from: a heteroatom; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered saturated or partially unsaturated heterocyclic group; and wherein the linking group is optionally further substituted.
7. The method of any one of aspects 2 to 6, wherein the linking group is attached to the solid support via a phosphoramidite group.
8. The method of any one of the preceding aspects, wherein the solid support comprises particles having a diameter of from about 10 to about 1000 µm.
9. The method of any one of the preceding aspects, wherein the solid support comprises a glass, ceramic, or a polymeric resin.
10. The method of any one of the preceding aspects, wherein Ⓝ is connected to the adjacent oxygen atom of formula (I) at the 3' position or the 5' position of the nucleoside, nucleotide, polynucleotide or derivative or analogue thereof.
11. The method of any one of the preceding aspects, wherein Ⓝ is represented by formula (N-1) or formula (N-2), 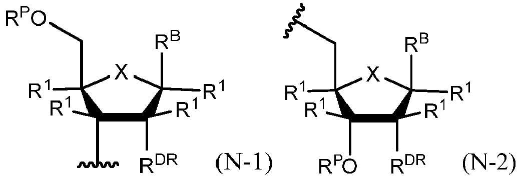 wherein:
   - X is an oxygen atom, a nitrogen atom, a sulphur atom or -C(R^{a}R^{a})-,
   - each R¹ and R^{DR} is independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a} and -C(O)R^{b}, wherein R^{a} and R^{b} are as defined in aspect 1, and when R^{DR} is -OR^{a} the R^{a} group and one of the R¹ groups may be joined together to form a bridging motif;
   - R^{P} is a hydrogen, hydroxyl protecting group, phosphoryl group or a salt or acid therof, a diphosphoryl group (-P(O₂⁻)-O-P(O₃⁻)) or a salt or acid thereof, a triphosphoryl group (-P(O₂⁻)-P(O₂⁻)-O-P(O₃⁻)) or a salt or acid thereof, or a phosphorous-based linkage to a nucleoside, nucleotide or polynucleotide or a derivative or analogue thereof,
   - R^{B} is an optionally substituted natural or non-natural nucleobase or a derivative or analogue thereof, and
   - the wavy line indicates the point of attachment to the compound of formula (I).
12. The method of aspect 11, wherein:
   - X is an oxygen atom; and/or
   - each R¹ is independently selected from hydrogen, halogen, C₁ to C₂ alkyl, C₁ to C₂ haloalkyl and C₁ to C₂ alkoxyl; preferably each R¹ is hydrogen; and/or
   - R^{DR} is a hydrogen or a halogen; preferably R^{DR} is hydrogen and/or
   - R^{P} is a hydrogen or a hydroxyl protecting group.
13. The method of any one of the preceding aspects, wherein step (i) comprises:
   (a) providing a compound of formula (Ia), 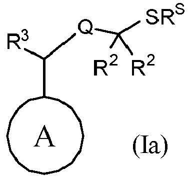 wherein Q, R², R³ and Ⓐ are as defined in any of aspects 1 to 9, and
      R^{S} is a C₁ to C₄ alkyl; and
   (b) reacting the compound of formula (Ia) with a nucleoside, nucleotide, polynucleotide or derivative or analogue thereof; wherein preferably the nucleoside, nucleotide, polynucleotide or derivative or analogue thereof is represented by formula (N-1) or formula (N-2) as defined in aspect 11 or aspect 12;
      thereby forming a compound of formula (I).
14. The method of any one of the preceding aspects, wherein step (i) comprises attaching a compound of formula (I) or formula (Ia) to a solid support, 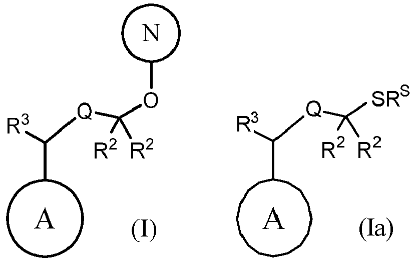 wherein Ⓝ, Q, R², R³, R^{S} and Ⓐ are as defined in any of the preceding aspects.
15. The method of any one of the preceding aspects, wherein increasing the chain length in step (ii) comprises performing the following step one or more times:
   contacting the support-attached nucleoside, nucleotide, polynucleotide, or derivative or analogue thereof with a nucleoside or a derivative thereof, thereby forming a phosphorous-based linkage.
16. The method of any one of the preceding aspects, wherein increasing the chain length in step (ii) comprises performing the following steps (a) to (e) one or more times:
   (a) providing a nucleoside having: (i) a phosphoramidite group at the 3' position and a hydroxyl protecting group at the 5' position; or (ii) a phosporamidite group at the 5' position and a hydroxyl protecting group at the 3' position;
   (b) activating the nucleoside phosphoramidite of step (a);
   (c) contacting the support-attached nucleoside, nucleotide, polynucleotide, or derivative or analogue thereof with the activated nucleoside phosphoramidite, thereby forming a phosphite triester linkage;
   (d) oxidising the phosphite triester linkage to form a phosphotriester linkage; and
   (e) removing the hydroxyl protecting group from the terminal nucleoside of the resulting support-attached polynucleotide.
17. The method of any one of the preceding aspects, wherein, after step (ii) and before step (iii), the method further comprises one or more steps of deprotecting the support-attached polynucleotide.
18. The method of any one of the preceding aspects, wherein step (iii) comprises photo-illuminating the compound of formula (I) using UV light at a wavelength of about 300 to about 500 nm.
19. A support-attached nucleoside, nucleotide, or derivative or analogue thereof, which is a compound of formula (I) attached to a solid support at Ⓐ, 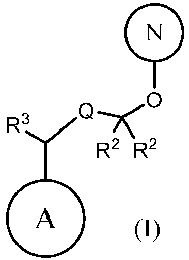 wherein Q, R², R³ and Ⓐ are as defined in any one of aspects 1 to 9,
   Ⓝ is a nucleoside, nucleotide or derivative or analogue thereof, wherein preferably the nucleoside, nucleotide, or derivative or analogue thereof is represented by formula (N-1) or formula (N-2) as defined in aspect 11 or aspect 12.
20. A compound of formula (I), 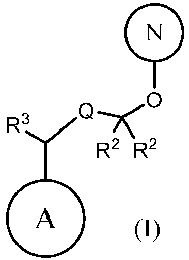 wherein Q, R², R³ and Ⓐ are as defined in any one of aspects 1 to 9, and
   Ⓝ is represented by formula (N-1) or formula (N-2) as defined in aspect 11 or aspect 12.
   In this aspect, for avoidance of doubt, Ⓐ may be optionally attached to a solid support; in other words, the solid support may be present or absent.
21. A support-attached compound of formula (Ia), which is attached to a solid support at Ⓐ, 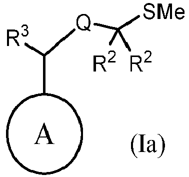 wherein Q, R², R³ and Ⓐ are as defined in any of the preceding aspects.
22. A method of producing a support-attached nucleoside, nucleotide or derivative or analogue thereof as defined in aspect 19, comprising:
   (a) providing a compound of formula (Ia) which is attached to a solid support at Ⓐ as defined in aspect 21; and
   (b) reacting the compound of formula (Ia) with a nucleoside, nucleotide or derivative or analogue thereof to produce a support-attached nucleoside, nucleotide or derivative or analogue thereof.
23. A solid support column for solid-phase polynucleotide synthesis, comprising a support-attached nucleoside, nucleotide, polynucleotide or a derivative or analogue thereof which is a compound of formula (I) attached to a solid support at Ⓐ, 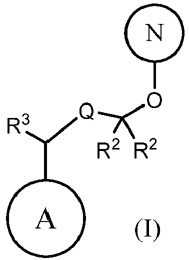 wherein Ⓝ, Q, R², R³ and Ⓐ are as defined in any one of the preceding aspects.
24. Use of a support-attached nucleoside, nucleotide or derivative or analogue thereof as defined in aspect 19, a compound of formula (I) as defined in aspect 20, or a compound of formula (Ia) as defined in aspect 21, for synthesising polynucleotides or derivatives or analogues thereof.
25. A polynucleotide or derivative or analogue thereof, obtainable by a method as defined in any one of aspects 1 to 18.

It is to be understood that although particular embodiments, specific configurations as well as materials and/or molecules, have been discussed herein for methods according to the present invention, various changes or modifications in form and detail may be made without departing from the scope and spirit of this invention. The following examples are provided to better illustrate particular embodiments, and they should not be considered limiting the application. The application is limited only by the claims.

### Examples

### Synthesis Example 1 - Synthesis of 1-(5-methyl-2-nitrophenyl)ethan-1-one

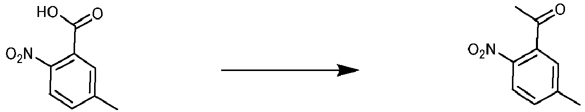

To a solution of 5-methyl-2-nitrobenzoic acid (5 g, 27.6 mmol) in anhydrous toluene (30 mL) was added thionyl chloride (6 mL, 82.8 mmol) and the mixture refluxed at 80 °C under argon for 3 hours. The mixture was concentrated to give 5-methyl-2-nitrobenzoyl chloride as a yellow oil (5.5 g) which was taken forward without further purification.

To a suspension of anhydrous magnesium chloride (2.36 g, 24.8 mmol) and diethyl malonate (5 mL, 33 mmol) in anhydrous ethyl acetate (80 mL) was added anhydrous triethylamine (16 mL) slowly at 0 °C. After stirring for 45 minutes, a solution of 5-methyl-2-nitrobenzoyl chloride (5.5 g) in anhydrous ethyl acetate (10 mL) was added. The mixture was the heated to 60 °C and stirred for 30 mins. Once complete, the reaction was extracted with ethyl acetate, washed with dilute hydrochloric acid (1 M), water, dried over anhydrous sodium sulfate, filtered and concentrated in *vacuo.* A solution of glacial acetic acid (30 mL) and conc. sulfuric acid (6 mL) was added and the mixture was refluxed for 12 hours. The reaction mixture was cooled in an ice bath and made alkaline with sodium hydroxide, the product extracted with dichloromethane before purification by flash chromatography (0-50% ethyl acetate in hexane) to give the title compound (3.79 g, 21.2 mmol, 77%).

¹H NMR (400 MHz, CDCl₃) δ ppm 8.03 (d, 1H, *J=* 8.4 Hz, CH Ar), 7.38 (m, 1H, CH Ar), 7.18 (m, 1H, CH Ar), 2.54 (s, 1H, CH₃CO), 2.48 (s, 1H, CH₃).

### Synthesis Example 2 - Synthesis of 1-(5-(bromomethyl)-2-nitrophenyl)ethan-1-one

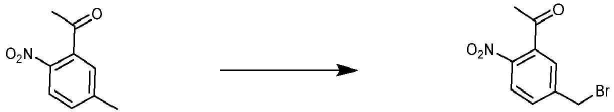

To a solution of 1-(5-methyl-2-nitrophenyl)ethan-1-one (3.79 g, 21.2 mmol) in anhydrous acetonitrile (25 mL) was added *N*-bromosuccinimide (4.14 g, 23.3 mmol) and 1,1'-Azobis(cyclohexanecarbonitrile) (0.52 g, 2.12 mmol) and the mixture refluxed for 12 hours. After cooling to room temperature, the solvent was removed and the residue dissolved in toluene (25 mL) and filtered. The filtrate was concentrated and purified by flash chromatography (0-50% ethyl acetate in hexane) to give the title compound (3.74 g, 14.5 mmol, 68%).

¹H NMR (400 MHz, CDCl₃) δ ppm 8.09 (d, 1H, *J=* 8.4 Hz, CH Ar), 7.62 (dd, 1H, *J =* 8.5, 2.0 Hz, CH Ar), 7.43 (d, 1H, *J =* 2.0 Hz, CH Ar), 4.50 (s, 2H, CH₂Br), 2.57 (s, 1H, CH₃CO).

### Synthesis Example 3 - Synthesis of 1-(5-(bromomethyl)-2-nitrophenyl)ethan-1-ol

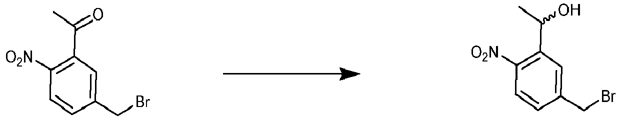

1-(5-(bromomethyl)-2-nitrophenyl)ethan-1-one (3.74 g, 14.5 mmol) was dissolved in mixture of anhydrous methanol (38 mL) and anhydrous dioxane (25 mL), whereupon sodium borohydride (0.822 g, 21.7 mmol) was added slowly at 0 °C, before allowing it to warm to room temperature over 12 hours. Water (60 mL) and 1M hydrochloric acid (15 mL) were added and the suspension extracted with ethyl acetate, dried over anhydrous sodium sulfate, filtered and concentrated before purification by flash chromatography (0-50% ethyl acetate in hexane) to give the title compound (3.38 g, 13.0 mmol, 90%).

¹H NMR (400 MHz, CDCl₃) δ ppm 7.91 (d, 1H, *J=* 8.4 Hz, CH Ar), 7.87 ppm (d, 1H, *J=* 2.0 Hz, CH Ar), 7.45 (m, 1H, CH Ar), 5.46 (q, 1H, *J=* 6.4 Hz, CH₃CH), 4.51 (s, 1H, CH₂Br), 2.27 (br. s, 1H, OH), 1.58 (d, 3H, *J=* 6.4 Hz, CH₃CH).

### Synthesis Example 4 - Synthesis of 1-(5-(azidomethyl)-2-nitrophenyl)ethan-1-ol

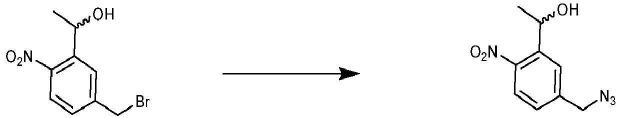

To a solution of 1-(5-(bromomethyl)-2-nitrophenyl)ethan-1-ol (2.49 g, 9.57 mmol) in anhydrous dimethyl formamide (30 mL) was added sodium azide (0.93 g, 14.4 mmol), and the mixture stirred for 3 hours at room temperature. The mixture was concentrated and then extracted from ethyl acetate with water, dried over anhydrous sodium sulfate, filtered and concentrated before purification by flash chromatography (0-50% ethyl acetate in hexane) to give the title compound (1.95 g, 8.77 mmol, 92%).

¹H NMR (400 MHz, CDCl₃) δ ppm 7.95 (d, 1H, *J=* 8.4 Hz, CH Ar), 7.81 (d, 1H, *J* = 2.0 Hz, CH Ar), 7.39 (dd, 1H, *J* = 8.4, 2.0 Hz, CH Ar), 5.48 (q, 1H, *J=* 6.4 Hz, CH₃CH), 4.49 (s, 2H, CH₂N₃), 2.17 (br. s, 1H, OH), 1.59 (d, 3H, *J=* 6.4 Hz, CH₃CH).

### Synthesis Example 5 - Synthesis of ((1-(5-(azidomethyl)-2-nitrophenyl)ethoxy)methyl) (methyl)sulfane

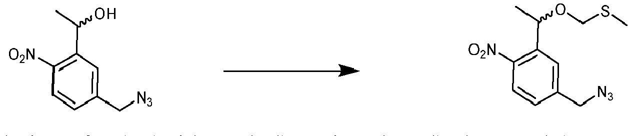

To a solution of 1-(5-(azidomethyl)-2-nitrophenyl)ethan-1-01 (1.65 g, 7.4 mmol) in DMSO (38 mL) was added acetic anhydride (57 mL) and glacial acetic acid (38 mL) and the reaction mixture was stirred at room temperature for 20 hours. The reaction mixture was poured into ice-cooled saturated aqueous sodium carbonate and stirred for a further 30 minutes and subsequently extracted with ethyl acetate. The combined organic layers were washed with saturated aqueous sodium carbonate, water and brine before drying over anhydrous sodium sulfate and concentrating. The crude was purified by flash chromatography (0-30% ethyl acetate in hexane), to give the title compound (1.7 g, 6 mmol, 81%).

¹H NMR (400 MHz, CDCl₃) δ ppm 7.96 (d, 1H, *J=* 8.4 Hz, CH Ar), 7.72 (d, 1H, *J* = 2.0 Hz, CH Ar), 7.39 (dd, 1H, *J =* 8.4, 2.0 Hz, CH Ar), 5.45 (q, 1H, *J=* 6.4 Hz, CH₃CH), 4.64 (d, 1H, *J=* 11.5 Hz, CH₂SCH₃), 4.49 (s, 2H, CH₂N₃), 4.35 (d, 1H, *J=* 11.5 Hz, CH₂SCH₃), 2.13 (s, 3H, CH₂SCH₃), 1.56 (d, 3H, *J* = 6.5 Hz, CH₃CH).

### Synthesis Example 6 - Synthesis of 3'-O-((1-(5-(azidomethyl)-2-nitrophenyl)ethoxy) methyl) thymidine

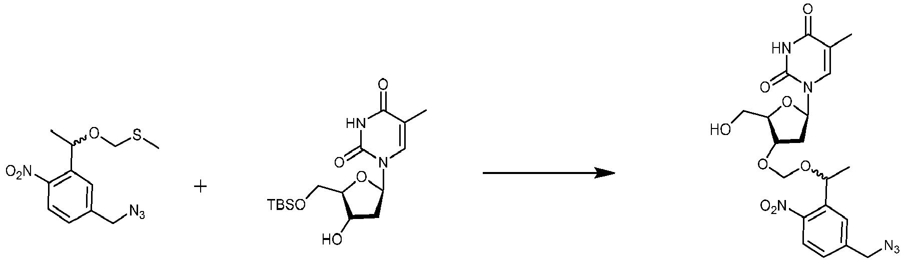

To a solution of ((1-(5-(azidomethyl)-2-nitrophenyl)ethoxy)methyl)(methyl)sulfane (1.7 g, 6 mmol) and 1-((2R,4S,5R)-5-(((tert-butyldimethylsilyl)oxy)methyl)-4-hydroxytetrahydrofuran-2-yl)-5-methylpyrimidine-2,4(1H,3H)-dione (2 g, 5.7 mmol) in anhydrous tetrahydrofuran (18 mL) at -40 °C was added *N*-iodosuccinimide (1.35 g, 6 mmol) and 4Å molecular sieves (1.8 g) followed by triflic acid (0.53 mL, 6 mmol) and the mixture stirred for 30 minutes. The reaction was quenched with triethylamine and filtered through celite before concentrating in *vacuo.* The residue was dissolved in ethyl acetate and washed with saturated aqueous sodium thiosulfate. The combined organic layers were washed with saturated sodium carbonate, water and brine before drying with anhydrous sodium sulfate, filtering and concentrating in *vacuo.* The residue was dissolved in anhydrous methanol (54 mL), ammonium fluoride (2.1 g, 57 mmol) was added and the mixture refluxed for 2 hours before cooling to room temperature for 12 hours. The reaction mixture was concentrated in *vacuo* and purified by flash chromatography (0-100% ethyl acetate in hexane), to give the title compound (1.2 g, 2.52 mmol, 42%).

¹H NMR (400 MHz, CDCl₃) δ ppm 8.66 (br. S., 1H, NH), 7.96 (d, 0.5H, *J=* 2.6 Hz, CHAr), 7.94 (d, 0.5H, *J=* 2.7 Hz, CHAr), 7.73-7.70 (m, 1H, CHAr), 7.42-7.32 (m, 2H, CHAr, CH⁶), 6.06 (t, 0.5H, *J=* 6.9 Hz, CH^{1'}), 6.01 (t, 0.5H, *J=* 6.9 Hz, CH^{1'}), 5.41-5.34 (m, 1H, CHCH₃), 4.77 (d, 0.5H, *J* = 7.3 Hz, OCH₂O), 4.74 (d, 0.5H, *J* = 7.2 Hz, OCH₂O), 4.6 (d, 0.5H, *J* = 7.2 Hz, OCH₂O), 4.57 (d, 0.5H, *J* = 7.3 Hz, OCH₂O), 4.52-4.45 (m, 2.5H, CH₂N₃, CH^{3'}), 4.38 (m, 0.5H, CH^{3'}), 4.09 (q, 0.5H, *J=* 2.9 Hz, CH^{4'}), 3.99-3.91 (m, 1H, CH^{4'}, CH^{5'}), 3.86-3.78 (m, 1H, CH^{5'}), 3.56-3.5 (m, 0.5H, CH^{5'}), 2.69 (br. S., 0.5H, OH), 2.59 (br. S., 1H, OH), 2.44-2.38 (m, 1H, CH^{2'}), 2.34-2.25 (m, 0.5H, CH^{2'}), 2.18-2.12 (m, 0.5H, CH^{2'}), 1.93-1.90 (m, 3H, CH₃), 1.57 (d, 3H, *J=* 6.4 Hz, CHCH₃).

### Synthesis Example 7 - Synthesis of 5'-O-(4,4'-dimethoxytrityl)-3'-O-((1-(5-(azidomethyl) -2-nitrophenyl)ethoxy)methyl) thymidine

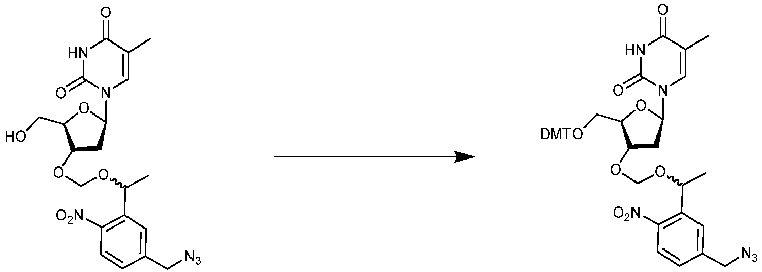

3'-O-((1-(5-(azidomethyl)-2-nitrophenyl)ethoxy)methyl) thymidine (1.2 g, 2.52 mmol) was co-evaporated with anhydrous pyridine three times before being dissolved in anhydrous pyridine (10 mL) under argon. 4,4'-dimethoxytrityl chloride (1 g, 3mmol) was added portion wise over 1 hour and the reaction stirred at room temperature for a further 11 hours. The reaction was quenched with methanol (5 mL) and triethylamine (1 mL) was added. The reaction was stirred for 10 min before the solvent was removed in *vacuo.* The crude product was purified *via* flash chromatography (0-100% ethyl acetate in (0.1% triethylamine in dichloromethane)) to give the title compound (1.85 g, 2.38 mmol, 94%).

¹H NMR (400 MHz, DMSO d6) δ ppm 11.35-11.34 (m, 1H, NH), 7.99-7.94 (m, 1H, CHAr), 7.70-7.67 (m, 1H, CHAr), 7.55-7.21 (m, 11H, CHAr, CH⁶, 9 x CH DMTr), 6.92-6.86 (m, 4H, CH DMTr), 6.13 (t, 0.5H, *J*= 7.0 Hz, CH^{1'} 6.10-6.04 (m, 0.5H, CH^{1'}), 5.23-5.10 (m, 1H, CHCH₃), 4.72 (d, 1H, *J=* 7.1 Hz, OCH₂O), 4.63-4.53 (m, 2.5H, CH₂N₃, OCH₂O), 4.5 (d, 0.5H, *J*= 7.5 Hz, OCH₂O) 4.44-4.36 (m, 1H, CH³'), 4.01-3.97 (m, 0.5H, CH^{4'}), 3.92-3.88 (m, 0.5H, CH^{4'}), 3.73 (s, 6H, 2 x OCH₃), 3.27-3.19 (m, 1H, CH^{5'}), 3.18-3.08 (m, 1H, CH^{5'}), 2.36-2.30 (m, 1H, CH^{2'}), 2.22-2.01 (m, 1H, CH^{2'}), 1.49-1.43 (m, 4.5H, CH₃, CHCH₃), 1.34 (d, 1.5H *J* = 6.4 Hz, CHCH₃).

### Synthesis Example 8 - Synthesis of 5'-O-(4,4'-dimethoxytrityl)-3'-O-((1-(5-((4-(4-hydroxybutyl)-1H-1,2,3-triazol-1-yl)methyl)-2-nitrophenyl)ethoxy)methyl) thymidine

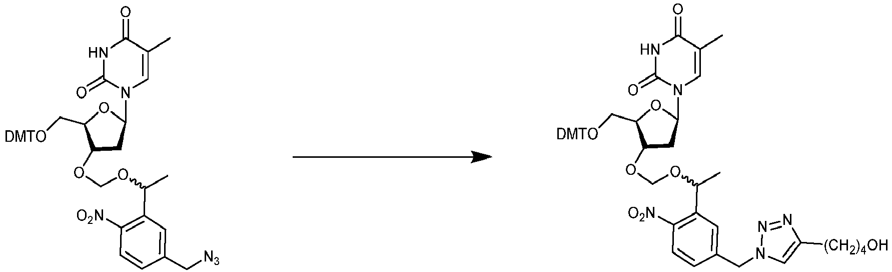

5'-O-(4,4'-dimethoxytrityl)-3'-O-((1-(5-(azidomethyl)-2-nitrophenyl)ethoxy)methyl) thymidine (0.7 g, 0.9 mmol) and 5-hexyn-1-ol (0.15 mL, 1.35 mmol) were added to a 3:1:1 mixture of tetrahydrofuran/*t*ert-butanol/water (13.5 mL/4.5 mL/4.5 mL). A 7.5% solution of copper(II) sulfate in water (2 mL) and a 1M solution of (+)-sodium L-ascorbate in water (2.25 mL) were added and the reaction stirred at room temperature for 2 hours. The mixture was diluted with ethyl acetate and the organic phase washed with saturated sodium bicarbonate and brine (diluted with water by 10%), dried over anhydrous sodium sulfate filtered and concentrated. The crude was purified by flash chromatography (0-10% methanol in (0.1% triethylamine in dichloromethane)) to give the title compound (0.7 g, 0.8 mmol, 59%).

¹H NMR (400 MHz, DMSO d6) δ ppm 11.35-11.34 (m, 1H, NH), 7.96-7.89 (m, 2H, CHAr, CH triazole), 7.61-7.59 (m, 1H, CH⁶), 7.5-7.47 (CHAr), 7.42-7.20 (m, 10H, CHAr, CH DMTr), 6.92-6.86 (m, 4H, CH DMTr), 6.16-6.04 (m, 1H, CH^{1'}), 5.67 (s, 1H, CH₂N), 5.63 (s, 1H, CH₂N), 5.17 (q, 0.5H, *J=* 6.4 Hz, CHCH₃), 5.1 (q, 0.5H, *J=* 6.3 Hz, CHCH₃), 4.67 (d, 1H, *J=* 7.2 Hz, OCH₂O), 4.51 (d, 0.5HJ= 7.2, OCH₂O), 4.45 (d, 0.5H, *J*= 7.3 Hz, OCH₂O), 4.43-4.33 (m, 2H, CH^{3'}, OH), 4.0-3.95 (m, 0.5H, CH^{4'}), 3.95-3.9 (m, 0.5H, CH^{4'}), 3.74-3.72 (m, 6H, 2 x OCH₃), 3.42-3.36 (m, 2H, CH₂OH), 3.28-3.20 (m, 1H, CH^{5'}), 3.19-3.09 (m, 1H, CH^{5'}), 2.59 (t, 2H, *J* = 7.6 Hz, CH₂CH₂ CH₂), 2.38-2.29 (m, 1H, CH^{2'}), 2.24-2.03 (m, 1H, CH^{2'}), 1.64-1.54 (m, 2H, CH₂CH₂ CH₂), 1.51-1.38 (m, 6.5H, CH₂CH₂ CH₂, CH₃, CH₃), 1.3 (d, 1.5H, *J* = 6.5 Hz).

### Synthesis Example 9 - Synthesis of 5'-O-(4,4'-dimethoxytrityl)-3'-O-((1-(5-((4-(4-[(2-cyanoethyl)(N,N-diisopropylamino)phosphino]oxybutyl)-1H-1,2,3-triazol-1-yl)methyl)-2-nitrophenyl)ethoxy)methyl) thymidine

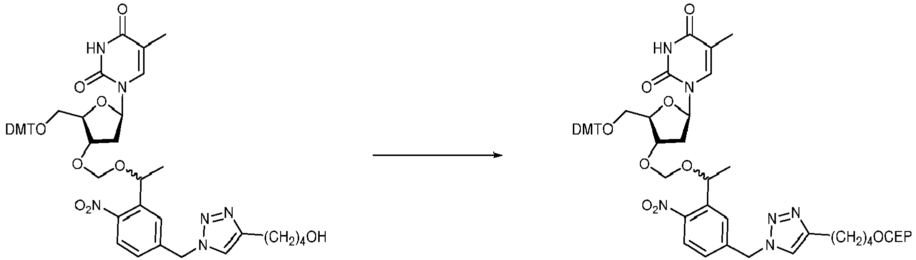

A solution of 5'-O-(4,4'-dimethoxytrityl)-3'-O-((1-(5-((4-(4-hydroxybutyl)-1H-1,2,3-triazol-1-yl)methyl)-2-nitrophenyl)ethoxy)methyl) thymidine (0.7 g, 0.8 mmol) in anhydrous dichloromethane (6 mL) was degassed under argon for 5 minutes before addition of anhydrous diisopropylethylamine (0.42 mL, 2.4 mmol). 2-Cyanoethoxy-*N,N-*diisopropylaminochlorophosphine (0.214 mL, 0.96 mmol) was added drop-wise and the reaction stirred at rt for 1 hour. The reaction mixture was diluted with anhydrous dichloromethane (2 x 20 mL) and washed with degassed saturated potassium chloride (30 mL), passed through anhydrous sodium sulfate, concentrated and dried under high vacuum. The crude product was purified by flash chromatography (100% 0.2% pyridine in ethyl acetate) under argon to obtain the title compound (0.61 g, 0.57 mmol, 71%, mix of diastereomers) as a colourless oil, which was dissolved in degassed anhydrous acetonitrile and filtered through a 0.45 µm syringe filter before concentrating and aliquoting.

¹H NMR (400 MHz, CD₃CN) δ ppm 8.96 (br. S., 1H, NH), 7.87 (d, 0.5H, *J* = 8.4 Hz, CH Ar), 7.84 (d, 0.5H, *J* = 8.3 Hz, CH Ar), 7.57 (d, 0.5H, *J* = 6.7 Hz, CH Ar), 7.56 (d, 0.5H, *J* = 6.2 Hz, CH Ar), 7.53 (s, 0.5H, CH triazole), 7.51 (s, 0.5H, CH triazole), 7.46-7.40 (m, 3H, CH⁶, 2 x CH DMTr), 7.35-7.22 (m, 8H, CH Ar, 7 x CH DMTr), 6.89-6.85 (m, 4H, 4 x CH DMTr), 6.14 (dd, 0.5H, *J* = 7.8 Hz, 6.1 Hz, CH^{1'}) 6.09 (dd, 0.5H, *J* = 7.6 Hz, 6.2 Hz, CH^{1'}), 5.55 (s, 1H, CH₂N), 5.51 (s, 1H, CH₂N), 5.24 (q, 0.5H, *J=* 6.5 Hz, CHCH₃), 5.17 (q, 0.5H, *J* = 6.4 Hz, CHCH₃), 4.65 (d, 0.5H, *J* = 7.2 Hz, OCH₂O), 4.64 (d, 0.5H, *J* = 7.3 Hz, OCH₂O), 4.5 (d, 0.5H, *J* = 7.4 Hz, OCH₂O), 4.46 (d, 0.5H, *J* = 7.3 Hz, OCH₂O), 4.42-4.36 (m, 1H, CH^{3'}), 4.00-3.98 (m, 0.5H, CH^{4'}), 3.91-3.89 (m, 0.5H, CH^{4'}) 3.79-3.52 (m, 6H, 2 x CH(CH₃)₂, CH₂CH₂CN, CH₂OP), 3.76 (s, 6H, 2 x OCH₃), 3.29 (dd, 1H, *J=* 3.67 Hz, 1.96 Hz, CH^{5'}), 3.18 (d, 1H, *J=* 3.55 Hz, CH^{5'}), 2.66 (t, 2H, *J* = 7.3 Hz, CH₂CH₂ CH₂), 2.69-2.59 (m, 2H, CH₂CH₂CN), 2.4-2.24 (m, 1H, CH^{2'}), 2.1-2.0 (m, 1H, CH^{2'}), 1.72-1.56 (m, 4H, CH₂CH₂ CH₂, CH₂CH₂ CH₂), 1.51 (d, 1.5H, *J* = 1.1 Hz, CH₃), 1.47 (d, 1.5H, *J=* 1.2 Hz, CH₃), 1.45 (d, 1.5H, *J=* 6.4 Hz, CHCH₃), 1.35 (d, 1.5H, *J=* 6.5 Hz, CHCH₃) 1.13 (dd, 12 H, *J* = 10.3 Hz, 6.8 Hz, 2 x CH(CH₃)₂). ³¹P {¹H} NMR (162 MHz, CD₃CN) δ ppm 148.3.

### UV cleavage studies

UV cleavage was performed using a handheld Analytik Jena UVP UVGL-25 4W UV lamp positioned roughly 2 cm from the sample. Irradiation at 365 nm for 20 minutes to a sample milli-Q water resulted in near quantitative cleavage.

Standard DNA phosphoramidites, solid supports and reagents were purchased from Link Technologies and Applied Biosystems. Automated solid phase synthesis of polynucleotides was performed on a K&A H-8 SE DNA/RNA synthesiser. Synthesis was performed on 0.2 µmole or 1.0 µmole scale involving cycles of acid-catalyzed detritylation, coupling, capping, and iodine oxidation. Standard DNA phosphoramidites were coupled for 60's. Coupling efficiencies and overall synthesis yields were determined by the inbuilt automated trityl cation conductivity monitoring facility and were ≥98.0% in all cases. The polynucleotides were then cleaved from the solid support on an Applied Biosystems 394 synthesiser with concentrated ammonium hydroxide for 60 minutes at room temperature followed by heating in a sealed tube for 5 h at 55° C to remove protecting groups from the nucleobase and backbone. Mass spectra of polynucleotides were recorded using a XEVO G2-QTOF MS instrument in ES⁻ mode. UV absorbance was performed on an Agilent Technologies Cary 60 UV-Vis in water at 260 nm.

### Example 1

A 21mer polynucleotide sequence (TTTTTTTTXTTTTTTTTTTTT, where X is the photocleaveable linker) was synthesised, cleaved from solid support and used without further purification. Mass calculated 6658.48, mass found 6659.0.

A 4mM solution of the polynucleotide in 1 mL milli-Q water was irradiated at 365 nm for 20 minutes and the results analysed by mass spec. The major product was the desired TTTTTTTTT strand released by the photocleavage mechanism. Mass calculated 2675.77, mass found 2675.6. The remaining material was composed of a series of polynucleotide strands containing the remains of the photocleavable linker. None of the starting 21mer sequence remained intact.

The experiment was repeated using the same sequence but without cleaving the polynucleotide from the solid support. Approximately 0.05 µmol worth of dried resin was placed in 1 mL milli-Q water and irradiated at 365 nm for 20 minutes. The solution was then passed through a 0.45 µm syringe filter before being analysed by mass spec. The only product found was the desired TTTTTTTTT strand, mass calculated 2675.77, mass found 2674.6. UV-Vis absorbance of the resulting solution showed the cleavage to be near quantitative.

## Claims

1. A method of producing a polynucleotide or an analogue or derivative thereof, comprising:
(i) providing a support-attached nucleoside, nucleotide, polynucleotide or derivative or analogue thereof, which is a compound of formula (I) attached to a solid support at Ⓐ, 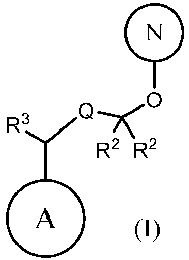 wherein:
- Ⓝ is a nucleoside, nucleotide, polynucleotide or derivative or analogue thereof,
- Q is an oxygen atom or a sulphur atom,
- each R² is independently selected from hydrogen, methyl, ethyl, C₁ to C₂ haloalkyl and a halogen group,
- R³ is methyl, ethyl or C₁ to C₂ haloalkyl, and
- Ⓐ is a 2-nitrobenzyl group, wherein said 2-nitrobenzyl group is optionally substituted with 1, 2 or 3 groups independently selected from halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, wherein each R^{a} is independently selected from hydrogen, optionally substituted C₁ to C₂ alkyl and optionally substituted C₁ to C₂ alkoxyl and each R^{b} is independently selected from hydrogen and an optionally substituted C₁ to C₄ alkyl group;
(ii) increasing the chain length of the support-attached nucleoside, nucleotide, polynucleotide or derivative or analogue thereof by introducing one or more additional nucleoside units, nucleotide units and/or analogues or derivatives thereof; and
(iii) cleaving the resulting polynucleotide from the solid support by photo-illuminating the compound of formula (I).

2. The method of claim 1, wherein
- Q is an oxygen atom, and/or
- each R² is independently selected from hydrogen, methyl, C₁ fluoroalkyl and fluorine; preferably each R² is hydrogen; and/or
- R³ is methyl, ethyl or C₁ to C₂ fluoroalkyl; preferably R³ is methyl; and/or
- Ⓐ is covalently attached to a linking group and the linking group is covalently attached to the solid support.

3. The method of claim 2, wherein Ⓐ is represented by formula (A-1) or formula (A-2), 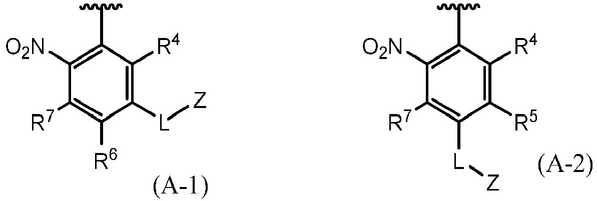 wherein:
- R⁴, R⁵, R⁶ and R⁷ are each independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a}, -C(O)R^{b}, -OC(O)R^{b} and -NHC(O)R^{b}, preferably R⁴, R⁵, R⁶ and R⁷ are each independently selected from hydrogen, fluorine, optionally substituted C₁ to C₄ alkyl, -OR^{a}, -SR^{a}, -NR^{a}R^{a}, -OC(O)R^{b} and -NHC(O)R^{b}, more preferably R⁴, R⁵, R⁶ and R⁷ are each hydrogen or methoxy; wherein R^{a} and R^{b} are as defined in claim 1;
- L is the linking group; and
- Z is the solid support.

4. The method of any one of claims 2 to 3, wherein the linking group comprises a C₁ to C₂₀ alkylene group, a C₂ to C₂₀ alkenylene group, a C₂ to C₂₀ alkynylene group and/or a nucleotide or a polynucleotide, wherein said alkylene, alkenylene or alkynylene group is optionally interrupted by and/or terminated in one or more groups selected from: a heteroatom; a phosphite group; a phosphate group; a carbonyl group; a C₆ to C₁₀ aryl group; a C₅ to C₁₀ carbocyclyl group; a 5- to 10-membered heteroaryl group; and a 5- to 10-membered saturated or partially unsaturated heterocyclic group; and wherein the linking group is optionally further substituted;
wherein preferably the linking group is attached to the solid support via a phosphoramidite group.

5. The method of any one of the preceding claims, wherein (i) the solid support comprises a glass, silica, ceramic, or a polymeric resin; and/or (ii) the solid support comprises particles having a diameter of from about 10 to about 1000 µm..

6. The method of any one of the preceding claims, wherein Ⓝ is connected to the adjacent oxygen atom of formula (I) at the 3' position or the 5' position of the nucleoside, nucleotide, polynucleotide or derivative or analogue thereof.

7. The method of any one of the preceding claims, wherein Ⓝ is represented by formula (N-1) or formula (N-2), 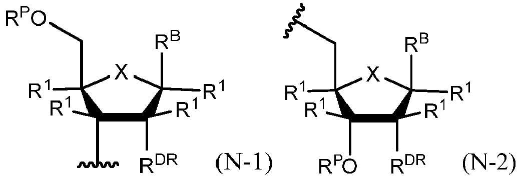 wherein:
- X is an oxygen atom, a nitrogen atom, a sulphur atom or -C(R^{a}R^{a})-,
- each R¹ and R^{DR} is independently selected from hydrogen, halogen, optionally substituted C₁ to C₄ alkyl, -OR^{a}, -SR^{a}, -NR^{a}R^{a}, -C(O)OR^{a}, -C(O)NR^{a}R^{a} and -C(O)R^{b}, wherein R^{a} and R^{b} are as defined in claim 1, and when R^{DR} is -OR^{a} the R^{a} group and one of the R¹ groups may be joined together to form a bridging motif;
- R^{P} is a hydrogen, hydroxyl protecting group, phosphoryl group or a salt or acid therof, a diphosphoryl group (-P(O₂⁻)-O-P(O₃⁻)) or a salt or acid thereof, a triphosphoryl group (-P(O₂⁻)-P(O₂⁻)-O-P(O₃⁻)) or a salt or acid thereof, or a phosphorous-based linkage to a nucleoside, nucleotide or polynucleotide or a derivative or analogue thereof,
- R^{B} is an optionally substituted natural or non-natural nucleobase or a derivative or analogue thereof, and
- the wavy line indicates the point of attachment to the compound of formula (I);
wherein more preferably:
- X is an oxygen atom; and/or
- each R¹ is independently selected from hydrogen, halogen, C₁ to C₂ alkyl, C₁ to C₂ haloalkyl and C₁ to C₂ alkoxyl; preferably each R¹ is hydrogen; and/or
- R^{DR} is a hydrogen or a halogen; preferably R^{DR} is hydrogen and/or
- R^{P} is a hydrogen or a hydroxyl protecting group.

8. The method of any one of the preceding claims, wherein step (i) comprises:
(a) providing a compound of formula (Ia), 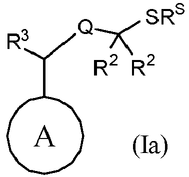 wherein Q, R², R³ and Ⓐ are as defined in any of claims 1 to 9, and
R^{S} is a C₁ to C₄ alkyl; and
(b) reacting the compound of formula (Ia) with a nucleoside, nucleotide, polynucleotide or derivative or analogue thereof; wherein preferably the nucleoside, nucleotide, polynucleotide or derivative or analogue thereof is represented by formula (N-1) or formula (N-2) as defined in claim 7;
thereby forming a compound of formula (I).

9. The method of any one of the preceding claims, wherein step (i) comprises attaching a compound of formula (I) or formula (Ia) to a solid support, 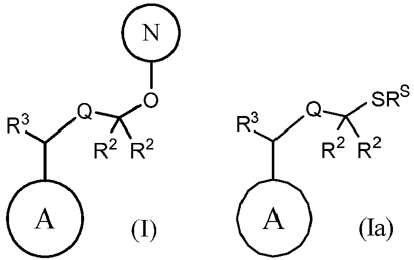 wherein Ⓝ, Q, R², R³, R^{S} and Ⓐ are as defined in any of the preceding claims.

10. The method of any one of the preceding claims, wherein increasing the chain length in step (ii) comprises performing the following step one or more times:
contacting the support-attached nucleoside, nucleotide, polynucleotide, or derivative or analogue thereof with a nucleoside or a derivative thereof, thereby forming a phosphorous-based linkage;
wherein increasing the chain length in step (ii) preferably comprises performing the following steps (a) to (e) one or more times:
(a) providing a nucleoside having: (i) a phosphoramidite group at the 3' position and a hydroxyl protecting group at the 5' position; or (ii) a phosporamidite group at the 5' position and a hydroxyl protecting group at the 3' position;
(b) activating the nucleoside phosphoramidite of step (a);
(c) contacting the support-attached nucleoside, nucleotide, polynucleotide, or derivative or analogue thereof with the activated nucleoside phosphoramidite, thereby forming a phosphite triester linkage;
(d) oxidising the phosphite triester linkage to form a phosphotriester linkage; and
(e) removing the hydroxyl protecting group from the terminal nucleoside of the resulting support-attached polynucleotide.

11. The method of any one of the preceding claims, wherein, after step (ii) and before step (iii), the method further comprises one or more steps of deprotecting the support-attached polynucleotide.

12. The method of any one of the preceding claims, wherein step (iii) comprises photo-illuminating the compound of formula (I) using UV light at a wavelength of about 300 to about 500 nm.

13. A support-attached nucleoside, nucleotide, or derivative or analogue thereof, which is a compound of formula (I) attached to a solid support at Ⓐ, 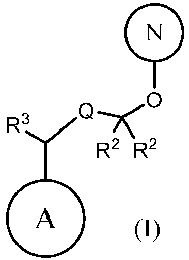 wherein Q, R², R³ and Ⓐ are as defined in any one of claims 1 to 9,
Ⓝ is a nucleoside, nucleotide or derivative or analogue thereof, wherein preferably the nucleoside, nucleotide, or derivative or analogue thereof is represented by formula (N-1) or formula (N-2) as defined in claim 11 or claim 12.

14. A compound of formula (I), 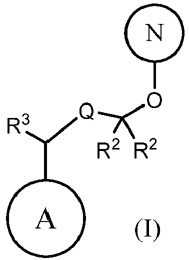 wherein Q, R², R³ and Ⓐ are as defined in any one of claims 1 to 5, and
Ⓝ is represented by formula (N-1) or formula (N-2) as defined in claim 7.

15. A support-attached compound of formula (Ia), which is attached to a solid support at Ⓐ, 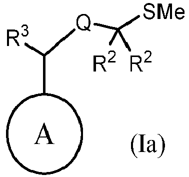 wherein Q, R², R³ and Ⓐ are as defined in any of the preceding claims.

16. A method of producing a support-attached nucleoside, nucleotide or derivative or analogue thereof as defined in claim 13, comprising:
(a) providing a compound of formula (Ia) which is attached to a solid support at Ⓐ as defined in claim 15; and
(b) reacting the compound of formula (Ia) with a nucleoside, nucleotide or derivative or analogue thereof to produce a support-attached nucleoside, nucleotide or derivative or analogue thereof.

17. A solid support column for solid-phase polynucleotide synthesis, comprising a support-attached nucleoside, nucleotide, polynucleotide or a derivative or analogue thereof which is a compound of formula (I) attached to a solid support at Ⓐ, 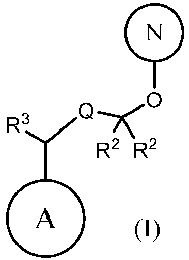 wherein Ⓝ, Q, R², R³ and Ⓐ are as defined in any one of the preceding claims.

18. Use of a support-attached nucleoside, nucleotide or derivative or analogue thereof as defined in claim 13, a compound of formula (I) as defined in claim 14, or a compound of formula (Ia) as defined in claim 15, for synthesising polynucleotides or derivatives or analogues thereof.

19. A polynucleotide or derivative or analogue thereof, obtainable by a method as defined in any one of claims 1 to 12.
